# EUROPEAN PATENT SPECIFICATION

(11) **EP 4 157 458 B1**
(45) Date of publication and mention of the grant of the patent: **17.04.2024**
(21) Application number: 21729865.2
(22) Date of filing: 28.05.2021
(51) Int. Cl.: A61P 35/00, C07D 401/14, A61K 31/4192

(54) **TRIARYLPYRIDINE COMPOUNDS AND USE THEREOF FOR TREATING CANCER**
TRIARYLPYRIDINVERBINDUNGEN UND IHRE VERWENDUNG ZUR BEHANDLUNG VON KREBS
COMPOSÉS DE TRIARYLPYRIDINE ET LEUR UTILISATION POUR LE TRAITEMENT DU CANCER

(30) Priority: 29.05.2020 EP 20305567
(43) Date of publication of application: 05.04.2023
(73) Proprietor: INSTITUT NATIONAL DE LA SANTE ET DE LA RECHERCHE MEDICALE (INSERM), 75013 Paris (FR); Centre National de la Recherche Scientifique (CNRS), 75016 Paris (FR); Institut Bergonié, 33000 Bordeaux (FR); UNIVERSITE DE BORDEAUX, 33000 Bordeaux (FR)
(72) Inventor: BEAUVARLET, Jennifer, 81675 Munich (DE); DJAVAHERI-MERGNY, Mojgan, 75006 Paris (FR); RABINDRA, Nath Das, 901 87 Umea (SE); MERGNY, Jean-Louis, 33076 Bordeaux (FR); GUILLON, Jean, 33076 Bordeaux (FR)
(74) Representative: Plasseraud IP
(86) International application number: PCT/EP2021/064405
(87) International publication number: WO 2021/239976

(56) References cited:
- N. M. SMITH ET AL: "Unraveling the relationship between structure and stabilization of triarylpyridines as G-quadruplex binding ligands", ORGANIC & BIOMOLECULAR CHEMISTRY, vol. 9, no. 17, 1 January 2011 (2011-01-01), page 6154, XP055717040, ISSN: 1477-0520, DOI: 10.1039/c1ob05560g cited in the application

## Description

### [TECHNICAL FIELD]

The present invention relates to the field of oncology, and more particularly relates to new triarylpyridine compounds, combination thereof with lysosomotropic agents, and their use for preventing and/or treating cancer diseases, in particular chemoresistant or potentially chemoresistant cancer diseases.

### [TECHNICAL BACKGROUND]

Cancer refers to a group of diseases characterized by the development of abnormal cells that divide uncontrollably and can infiltrate and destroy normal body tissue. Cancer is the second-leading cause of death in the world. Numerous therapies, in particular chemotherapies, have been developed to treat the various cancer diseases. However, sometimes, cancer cells can overcome the efficacy of the anticancer agents and may become chemoresistant.

Mounting evidences indicate that lysosomes are involved in resistance to anticancer agents. One proposed mechanism is the sequestration of the anticancer agent into the lysosome which results in reduction of the accessibility of the drug to its cellular targets as well as of its effectiveness. This effect ultimately has been suggested to contribute to resistance to chemotherapy (Gong Y et al., J Pharmacol Exp Ther. 2006; 316: 242-247; Herlevsen et al., Mol Cancer Ther. 2007; 6: 1804-1813; Smith et al., Cancer Res. 1992; 52: 4000-4008; Zhitomirsky et al., Oncotarget 2015; 6: 1143-1156; Kroemer et al., Oncotarget 2017; 8: 112168-112169).

G-quadruplexes (G4s) are four-stranded non-canonical nucleic acids secondary structures which form in guanine-rich DNA and RNA sequences. Many G-quadruplex forming sequences are found to be associated with cancer. Some compounds, named G-quadruplex interactive ligands (or G-quadruplex ligands, or G4 ligands, abbreviated as G4L) have been shown to strongly interact with G-quadruplex DNA and to inhibit cancer proliferation (Smith et al., Org Biomol Chem. 2011; 9(17):6154-62). Those G-quadruplex ligands were proposed as anticancer agents based on their antiproliferative and chemosensitizing effects against tumor models both *in vitro* and *in vivo* (Li Q et al., Current Pharmaceutical Design 2012; 18, 1973-1983; Asamitsu S et al., Molecules 2019; 254:429).

Despite many G4L have been described to inhibit cancer proliferation, cancer remain life threatening and lead to severe sequelae.

Therefore, there remains a need to set up new and improved compounds for treating cancer.

There is a need to provide new and improved compounds which display an improved ability to induce cancer cells death than known anticancer G-quadruplex ligands, towards the cancer target cells.

There is a need to provide new compounds, in particular new triarylpyridine compounds, with improved efficacy towards the target cancer cells.

There is a need to provide new combinations of anticancer active agents with improved efficacy, in particular with synergistic efficacy, towards the target cancer cells.

There is a need to provide new compounds with a lysosomotropic effect.

There is a need to provide new compounds able to efficiently trigger lysosomal membrane permeabilization in target cancer cells.

There is a need to provide new compounds and combinations of active agents with increased efficacy towards lysosomal membrane permeabilization-induced cancer cells death.

Lysosomal dependent non-apoptotic cell death is of tremendous interest in cancer therapy as cancer cells often display defective apoptotic machinery rendering them resistant to therapy.

The present invention has for purpose to satisfy all or part of those needs.

### [SUMMARY]

Any references hereinafter to methods of treatment refer to the compounds, pharmaceutical compositions and medicaments of the present invention for use in a method for treatment of the human (or animal) body by therapy."

According to a first embodiment, the invention is directed to a compound of general formula (I): 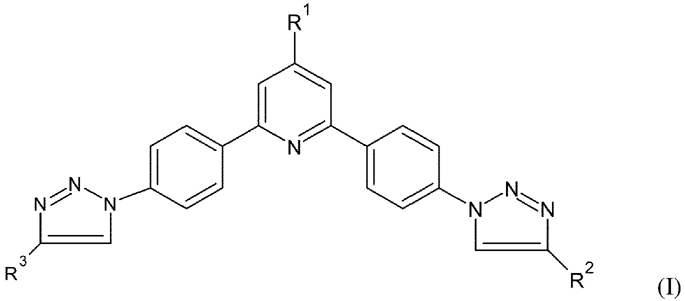 wherein
- R¹ is a 5 to 6 membered unsaturated cycle, optionally comprising at least one heteroatom, and optionally substituted with either at least one:
   - C₁-C₄ saturated or unsaturated, linear or branched, alkyl group;
   - Y-R⁴ group with Y being N, O or S and R⁴ being either a C₁-C₄, saturated or unsaturated, linear or branched, alkyl group, or a benzyl group; or
   - halogen atom selected among F, Cl or Br;
- R² and R³, different or identical, in particular identical, are each -(CH₂)ₙ-Het, with
   - n ranging from 1 to 4, in particular from 2 to 3, and
   - Het being a saturated or unsaturated 5 to 6 membered heterocycle group, optionally substituted with at least one C₁-C₄ saturated or unsaturated, linear or branched, alkyl group;
   or a racemate, enantiomer, diastereoisomer of such compound or mixture thereof, or an addition salt of such compound, racemate, enantiomer, diastereomer or mixture with a mineral acid or organic acid.

As shown in the examples, it has been observed that the new compounds of the invention are able to induce cancer cell death, and in particular to induce lysosomal membrane permeabilization-induced cancer cells death. The compounds of the invention display improved activity compared to the known triarylpyridine **20A** (compound n°3 in Smith et al., Org. Biomol. Chem. 2011). Further, and more advantageously, the new compounds of the invention are able to synergistically act with a lysosomotropic agent, such as chloroquine or chloroquine diphosphate, to induce cancer cell death. This synergistic effect is even improved compared to effect obtained with a combination of **20A** and chloroquine.

In one embodiment, the compounds of the invention may be of general formula (I) wherein:
- R¹ is a 5 to 6 membered unsaturated cycle, optionally comprising at least one O or S, and optionally substituted with at least one group being:
   - Y-R⁴ with Y being O or S and R⁴ being either a C₁-C₃, saturated or unsaturated, linear or branched, alkyl group or a benzyl group; or
   - Cl or Br;
- R² and R³, different or identical, in particular identical, are each -(CH₂)ₙ-Het, with
   - n being 1, 2 or 3, and
   - Het being a saturated or unsaturated 5 to 6 membered nitrogen heterocycle group, optionally substituted with at least one C₁-C₃ saturated or unsaturated, linear or branched, alkyl group.

In one embodiment, R¹ may be either an unsubstituted, unsaturated 5 to 6 membered cycle, comprising one O or S, or may be a phenyl group substituted with at least one group selected among Y-R⁴, Cl or Br, with Y being O or S and R⁴ being either a C₁-C₃, saturated or unsaturated, linear or branched, alkyl group or a benzyl group.

In another embodiment, R¹ may be selected among a furyl; a thienyl; or a phenyl substituted with at least one group selected among Y-R⁴, Cl or Br, wherein Y is O or S and R⁴ is either a C₁-C₂, saturated or unsaturated, alkyl group or a benzyl group.

In another embodiment, R¹ may be a phenyl substituted with at least one group being Y-R⁴ or Br, with Y being O or S and R⁴ being either a methyl or a benzyl group.

In another embodiment, R¹ may be a phenyl substituted with at least one group being S-CH₃, O-benzyl, or Br, and in particular being S-CH₃.

In another embodiment, the phenyl is substituted with a single substituting group, in particular in position 4 of the phenyl.

In another embodiment, in the compounds of the invention as above defined, R² and R³ may be identical.

In another embodiment, in the compounds of the invention as above defined, n may be 2 or 3.

In another embodiment, in the compounds of the invention as above defined, Het may be a saturated 5 to 6 membered nitrogen heterocycle group comprising one or two nitrogen atoms.

In one embodiment, Het may be selected among a pyrrolidinyl, a piperidinyl, or a piperazinyl group.

In another embodiment, Het may be unsubstituted or may be substituted with at least one C₁-C₂, saturated or unsaturated, alkyl group, in particular with at least one methyl group.

In another embodiment, Het may be unsubstituted or may be substituted with one methyl group.

In another embodiment, Het may be either an unsubstituted pyrrolidine or piperidine or may be a piperazine substituted with a methyl group in position 4.

In another embodiment, a compound of the invention may be selected from the group consisting of formulas (IIa), (IIb), (IIc), (IId) or (IIe):

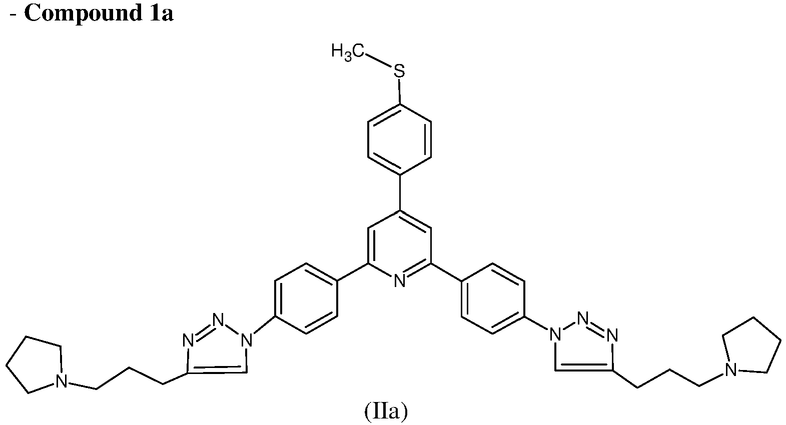

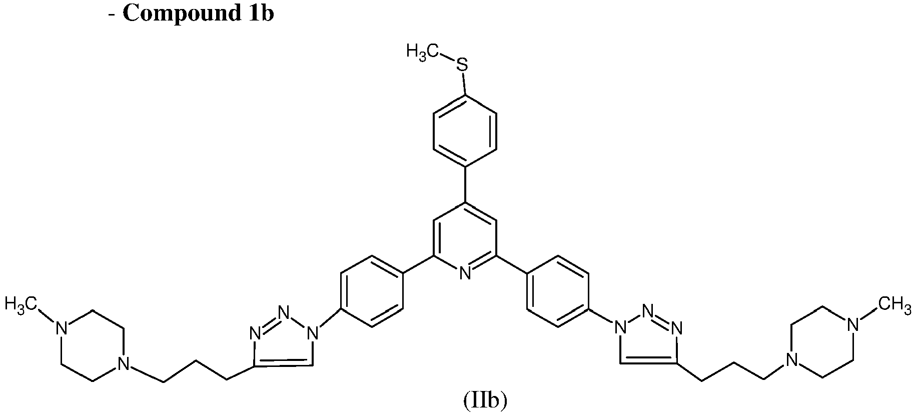

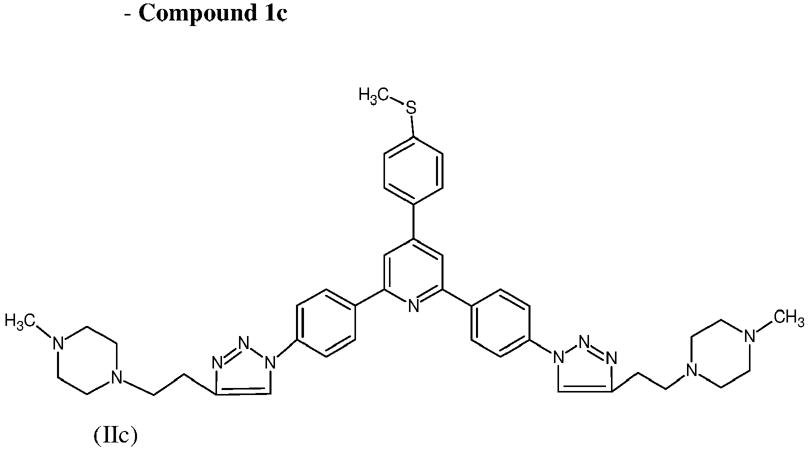

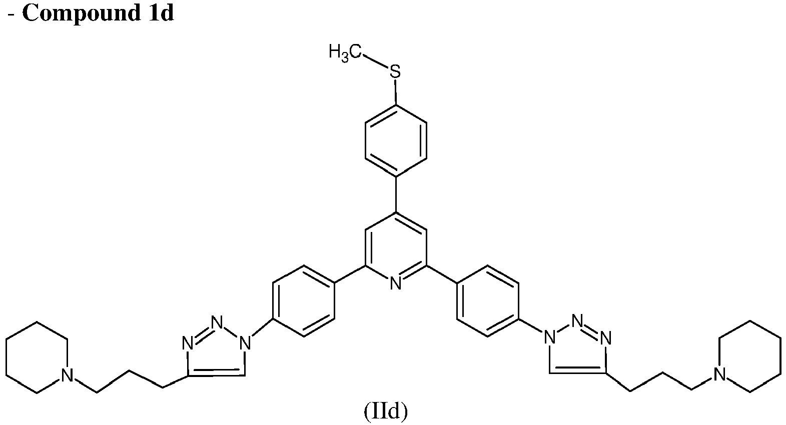

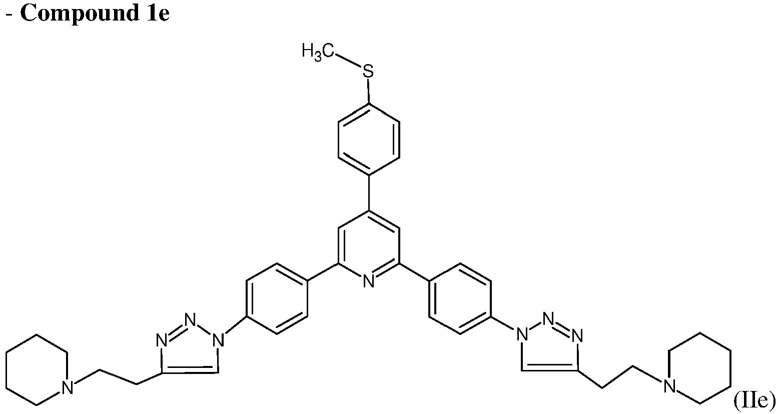

According to another object, the invention is directed to a combination comprising (i) at least one compound of the invention, and (ii) at least one lysosomotropic agent.

In one embodiment, in a combination of the invention, the lysosomotropic agent may be selected in the group comprising: chloroquine and derivatives thereof; Lys05; siramesine; GNS561; nanaomycin; siomycin A; helenalin; or a pharmaceutically acceptable salt thereof.

In another embodiment, in a combination of the invention, the lysosomotropic agent may be chloroquine; Lys05; or a pharmaceutically acceptable thereof, and in particular is chloroquine diphosphate or Lys05.

According to another object, the invention is directed to a pharmaceutical composition comprising (i) at least one compound of the invention or at least one combination of the invention, and (ii) a pharmaceutically acceptable excipient or carrier.

According to another object, the invention is directed to kit-of-parts comprising at least a first and a second containers, (i) the first container containing at least a first composition comprising at least one compound of the invention, and (ii) the second container containing at least a second composition comprising at least one lysosomotropic agent.

According to another object, the invention is directed to a compound of the invention, for use as a medicament.

According to another object, the invention is directed to a compound of the invention, a combination of the invention, a pharmaceutical composition of the invention, or a kit-of-parts of the invention, for use in a therapeutic method for preventing and/or treating a cancer disease.

According to one embodiment, the cancer disease may be a chemoresistant cancer disease.

According to another embodiment, the cancer disease may be selected from the group consisting of: breast cancer; colon cancer; rectal cancer; endometrial cancer; gastric carcinoma; glioblastoma; hepatocellular carcinoma; cervical carcinoma; lung adenocarcinoma; melanoma; medulloblastoma; ovarian carcinoma; osteosarcoma; pancreatic cancer; prostate cancer; acute myelogenous leukemia; chronic myelogenous leukemia; non-Hodgkin's lymphoma; thyroid carcinoma; and pediatric tumors.

According to another embodiment, a compound of the invention and a lysosomotropic agent in a combination of the invention, or in a pharmaceutical composition of the invention, or in a kit-of-parts of the invention may be for simultaneous, separate or sequential use.

### [BRIEF DESCRIPTION OF THE FIGURES]

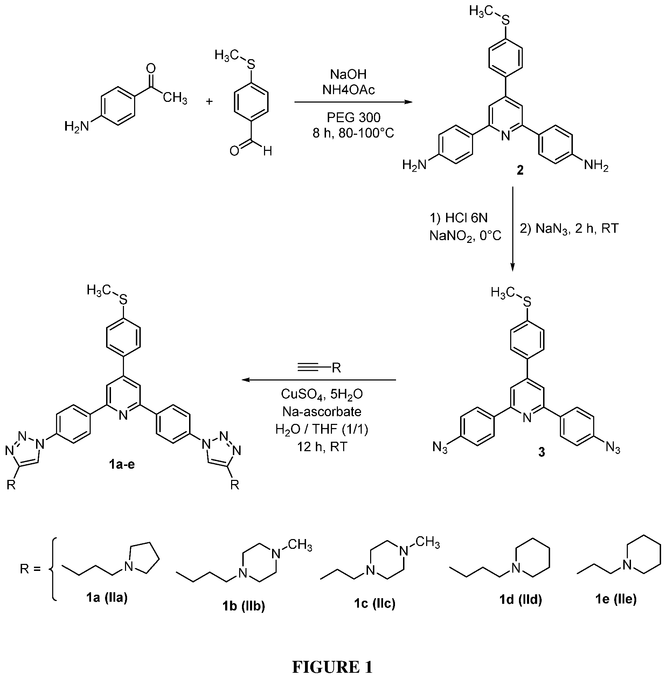

**Figure 1** shows a scheme for synthesis the three bis-triazole 2,4,6-triarylpyridines compounds (**1a**, **1b** and **1c**). The synthetic route involves the base-catalysed condensation of commercially available p-aminoacetophenone (1) with p-thiomethyl benzaldehyde (2) in PEG300 resulting the formation of di-amino derivative of 2,4,6- triarylpyridines (3). The di-amino (3) is then converted to corresponding diazido derivatives (4) which are then subjected for copper-catalyzed azide-alkyne 1,3-dipolar cycloaddition reaction with various amine-terminal alkynes to afford 1,2,3-triazole derivatives (**1a**, **1b** and **1c**).

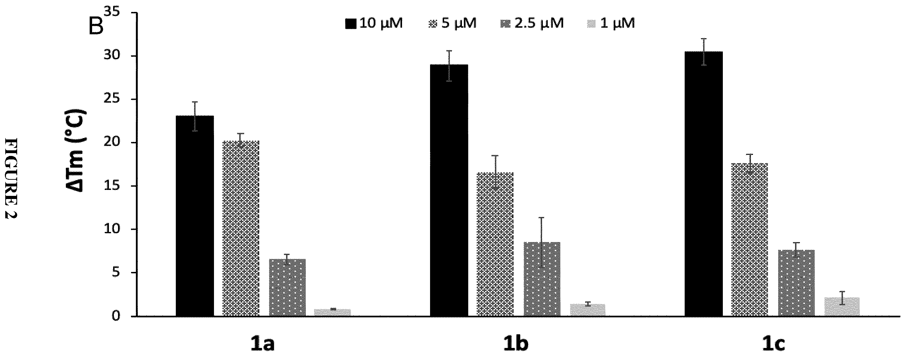

**Figure 2** shows that the compounds **1a, 1b** and **1c** stabilize an intramolecular quadruplex and confirms the G4 recognition. Compounds **1a, 1b** and **1c** were tested at (i) 1µM (grey bars), (ii) 2.5µM (white-dotted grey bar), (iii) 5µM (black-dotted grey bars) and (iv) 10µM (black bars) concentration in the presence of the F21T oligonucleotide (intramolecular quadruplex) (FAM-5'-GGGTTAGGGTTAGGGTTAGGG-3'-TAMRA) for 1 h. The stabilization of the F21T oligonucleotide by the compounds **1a, 1b** and **1c** was assessed by evaluating the difference in the mid-transition temperature using FRET melting analysis. Ordinate: melting temperature (Tₘ) in Celsius scale (°C). Abscissa (from left to right): (i) Compounds **1a** at 10µM, 5µM, 2.5µM, 1µM; (ii) Compounds **1b** at 10µM, 5µM, 2.5µM, 1µM; (iii) Compounds **1c** at 10µM, 5µM, 2.5µM, 1µM.

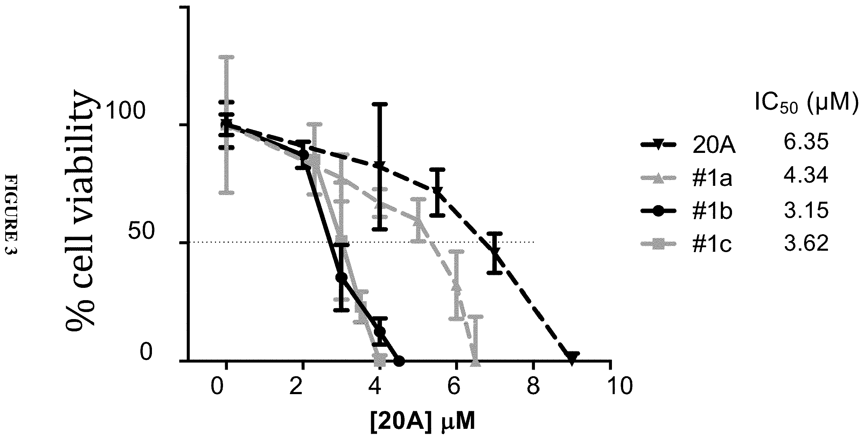

**Figure 3** shows a representative experiment performed 4 times in quadruplicate evaluating the HeLa cells viability after treatment with compounds **20A, 1a, 1b** and **1c**. HeLa cells were treated with 1µM to 10µM concentrations of compounds 20A (▼), 1a (grey ▲), 1b (●) and 1c (■) for 24h. Cell viability was assessed by the MMT assay. Plotted are mean ± SD of one representative experiment each performed in quadruplicate. IC₅₀ is scored for each compound from 4 independent experiments performed in quadruplicate. Ordinate: Percentage of HeLa cell viability. Abscissa: Concentrations of compounds **20A, 1a, 1b** and **1c.**

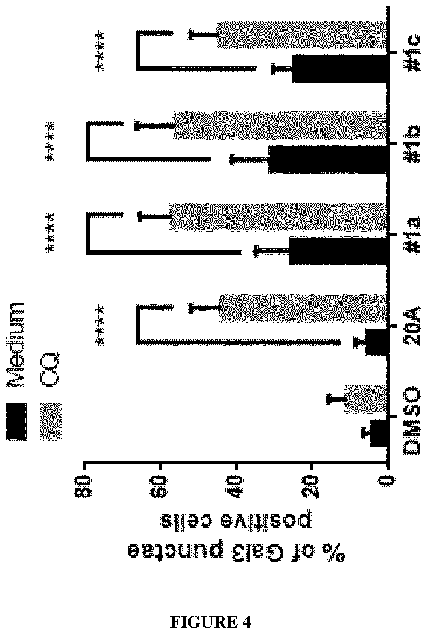

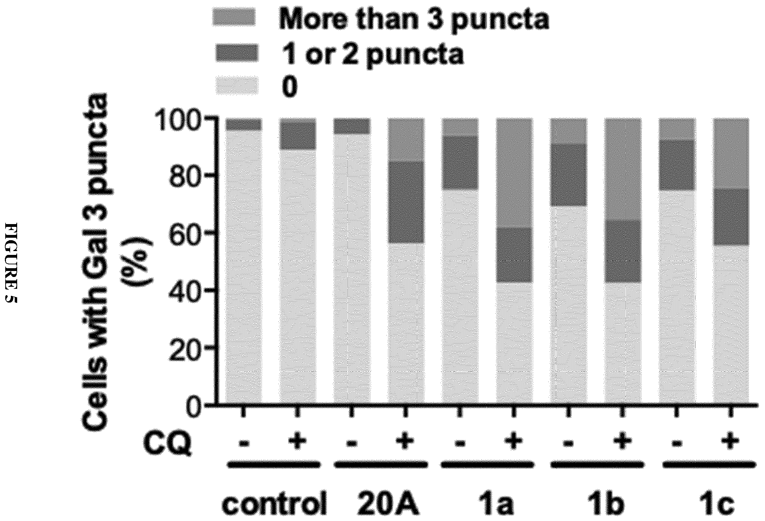

**Figures 4** and **5** show that compounds **20A, 1a, 1b** and **1c** with or without chloroquine allow to trigger lysosomal membrane permeabilization (LMP) in cancer cells U2OS expressing Galectin3-mcherry.
**Figure 4** shows the percentage of cells displaying at least one Galectin-3 puncta in presence of DMSO (negative control), 3 µM of **20A,** 2 µM of **1a,** 1 µM of **1b** and 1 µM of **1c,** with (grey bar + CQ) or without (black bar - CQ) 25 µM of chloroquine for 24 h. The data are presented as the mean percentage of 15 values from 5 randomly chosen area in each of the three independent experiments. Ordinate: Percentage of cells with Galectin3 puncta. Abscissa (from left to right): (i) DMSO without or with chloroquine; (ii) **20A** without or with chloroquine; (iii) **1a** without or with chloroquine; (iv) **1b** without or with chloroquine; **1c** without or with chloroquine. **** *p-value* < 0.0001 using Mann-Whitney test.
**Figure 5** shows the percentage of cells > 3, 1 or 2, 0 Galectin-3 puncta (0) puncta - light grey; 1 or 2 puncta - medium grey; more than 3 puncta - dark grey). Data are presented as the mean percentage of at least 1000 cells analyzed from 5 randomly chosen field in each of the three independent experiments. Ordinate: Percentage of cells displaying with Galectin 3 puncta. Abscissa (from left to right): (i) DMSO (negative control) without or with chloroquine (CQ); (ii) Compound 20A (positive control) without or with chloroquine (CQ); (iii) Compound **1a** without or with chloroquine (CQ); (iv) Compound **1b** without or with chloroquine (CQ); (v) Compound **1c** without or with chloroquine (CQ). **** *p-value* < 0.0001 using Mann-Whitney test.

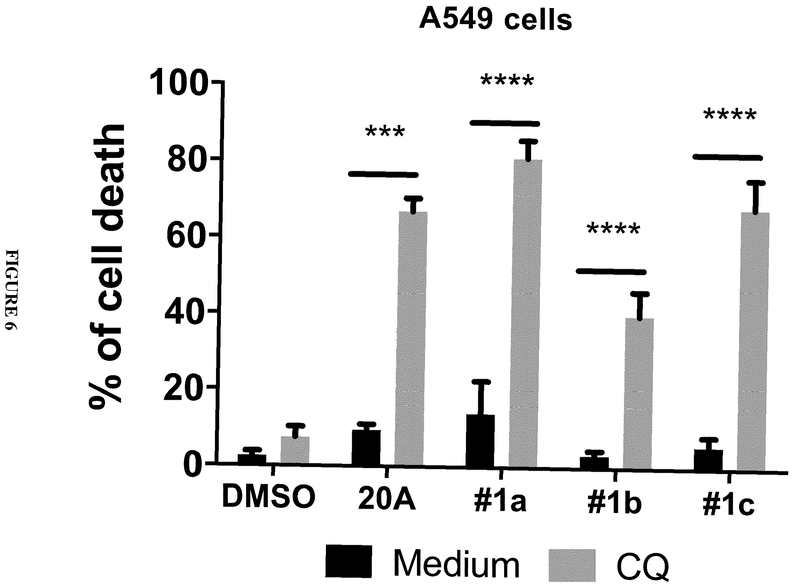

**Figure 6** shows that combination of chloroquine with compounds **1a, 1b** and **1c** significantly enhances cell death in A549 lung cancer cells. Cell death was evaluated by scoring the percentage of PI-positive cells by flow cytometer analysis. The data represents the mean ± SD of 9 values obtained from three independent experiments each performed in triplicate. *p-value* *** < 0.001 and **** < 0.0001 using Mann-Whitney test. A549 cells were treated with either DMSO (negative control), 3.5µM of **20A** (positive control), 2.5µM of **1a,** 1.5µM of **1b** or 2µM of **1c,** in either presence (CQ) or absence of 25 µM chloroquine for 24h. Ordinate: Percentage of cell death. Abscissa (left to right): (i) DMSO without or with chloroquine; (ii) **20A** without or with chloroquine; (iii) **1a** without or with chloroquine; (iv) **1b** without or with chloroquine; (v) **1c** without or with chloroquine.

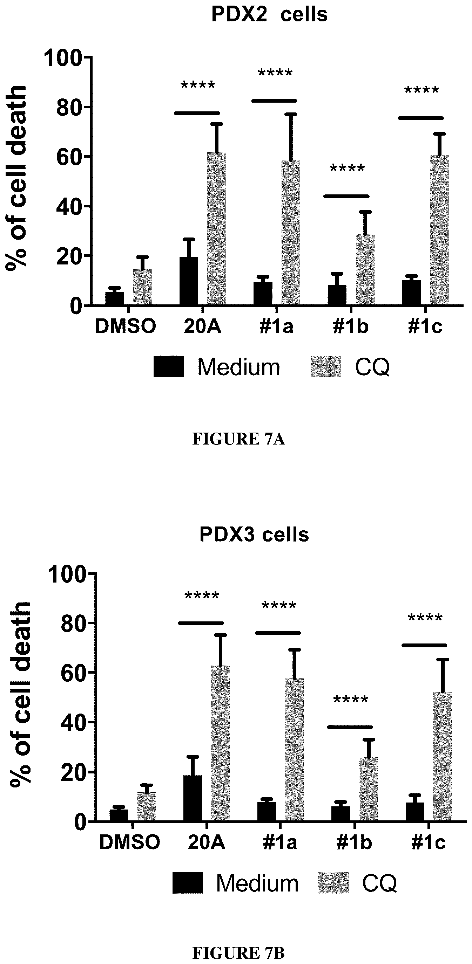

**Figures 7A and 7B** show that combination of chloroquine with compounds **1a, 1b** and **1c** significantly enhances cell death in patient-derived xenograft cell lines from lung cancer. Cell death was evaluated by scoring the percentage of PI-positive cells by flow cytometer analysis. The data represents the mean ± SD of 9 values obtained from three independent experiments each performed in triplicate. p-value *** < 0.001 and **** < 0.0001 using Mann-Whitney test. Two PDX from lung cancer PDX2 cells (**Fig. 7A**) and PDX3 cells (**Fig. 7B**) were treated with either DMSO (negative control), 3.5µM of **20A** (positive control), 2.5µM of **1a,** 1.5µM of **1b** or 2µM of **1c**, in either presence (CQ) or absence (Medium) of 25 µM chloroquine for 24h. Ordinate: Percentage of cell death. Abscissa (left to right): (i) DMSO without or with chloroquine; (ii) **20A** without or with chloroquine; (iii) **1a** without or with chloroquine; (iv) **1b** without or with chloroquine; (v) **1c** without or with chloroquine.

### [DETAILED DESCRIPTION]

### Definitions

As used in this specification and the appended claims, the singular forms "**a**", "**an**" and "**the**" include plural referents unless the content clearly dictates otherwise.

The term "**about**" or "**approximately**" as used herein refer to the usual error range for the respective value readily known to the skilled person in this technical field. Reference to "about" a value or parameter herein includes (and describes) embodiments that are directed to that value or parameter per se. In some embodiments, the term "about" refers to ±10% of a given value. However, whenever the value in question refers to an indivisible object, such as a nucleotide or other object that would lose its identity once subdivided, then "about" refers to ±1 of the indivisible object.

It is understood that aspects and embodiments of the present disclosure described herein include "**comprising**," "**having**," "**consisting of**," and "**consisting essentially of"** aspects and embodiments. The words "have" and "comprise," or variations such as "has," "having," "comprises," or "comprising," will be understood to imply the inclusion of the stated element(s) (such as a composition of matter or a method step) but not the exclusion of any other elements. The term "consisting of' implies the inclusion of the stated element(s), to the exclusion of any additional elements. The term "consisting essentially of" implies the inclusion of the stated elements, and possibly other element(s) where the other element(s) do not materially affect the basic and novel characteristic(s) of the invention. Depending on the context, the term "**comprise**" may also specify strictly the stated features, integers, steps or components, and therefore in such case it may be replaced with "**consist of".**

Within the invention, "**derivative(s)**" with respect to a particular compound or a particular family of compounds is intended to mean a compound or a set of compounds presenting substantially the same chemical structure for substantially the same biological or pharmacological activity. The chemical structure of a derivative according to the invention may differ from the chemical structure of a reference compound (i) by the absence of one or more substituents and/or (ii) the presence of one or more supplemental substituents and/or (iii) the substitution of one or more substituents by another having substantially the same physico-chemical properties, for instance a fluorine by a chlorine or a methyl by an ethyl, in such a manner that the derivative has substantially the same biological or pharmacological activity than the reference compound.

As used herein, the term **"individual"** or "**subject**" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In some embodiments, the individual or subject is a human.

As used herein, a **"pharmaceutically acceptable salt**" of a compound or of an agent in accordance with the invention means a salt that is pharmaceutically acceptable and that possesses the desired pharmacological activity of the parent agent. It is understood that the pharmaceutically acceptable salts are non-toxic. As used herein, the term "**salt**" refers to acid salts of agents used in the present invention. Illustrative examples of acceptable salts are mineral acid (hydrochloric acid, hydrobromic acid, phosphoric acid, and the like) salts, organic acid (acetic acid, propionic acid, glutamic acid, citric acid and the like) salts, quaternary ammonium (methyl iodide, ethyl iodide, and the like) salts. The neutral forms of the compounds are in particular regenerated by contacting the salt with an acid and isolating the parent agent in the conventional manner. The parent form of the agent differs from the various salt forms in certain physical properties, such as solubility in polar solvents.

The term "**acid addition salts**" include inorganic acids such as hydrochloric, hydrobromic, hydroiodic, sulfuric, nitric and phosphoric acid, as well as organic acids such as acetic, citric, propionic, tartaric, glutamic, salicylic, oxalic, methanesulfonic, paratoluenesulfonic, succinic, and benzoic acid, and related inorganic and organic acids.

As used herein, the terms "**prevent**", "**preventing**" or "**delay progression of"** (and grammatical variants thereof) with respect to a disease or disorder relate to prophylactic treatment of a disease, *e.g.,* in an individual suspected to have the disease, or at risk for developing the disease. Prevention may include, but is not limited to, preventing or delaying onset or progression of the disease and/or maintaining one or more symptoms of the disease at a desired or sub-pathological level. The term "prevent" does not require the 100% elimination of the possibility or likelihood of occurrence of the event. Rather, it denotes that the likelihood of the occurrence of the event has been reduced in the presence of a composition or method as described herein. More particular, "prevent" or "preventing" refers to a decrease in the risk of occurrence of a cancer disease or symptom in a patient. As indicated above, the prevention may be complete, i.e. no detectable symptoms or disease, or partial, such that fewer symptoms or less severity of the disease are observed than would likely occur absent treatment.

Within the invention, the term "**significantly**" used with respect to change intends to mean that the observe change is noticeable and/or it has a statistic meaning.

Within the invention, the term "**substantially**" used in conjunction with a feature of the invention intends to define a set of embodiments related to this feature which are largely but not wholly similar to this feature.

The terms "**treat**" or "**treatment**" or "**therapy**" in the present text refers to the administration or consumption of a compound or composition according to the invention with the purpose to cure, heal, alleviate, relieve, alter, remedy, ameliorate, improve, or affect a disorder, the symptoms of the condition, or to prevent or delay the onset of the symptoms, complications, or otherwise arrest or inhibit further development of the disorder in a statistically significant manner. More particularly, "treating" or "treatment" includes any approach for obtaining beneficial or desired results in a subject's cancer condition. Beneficial or desired clinical results can include, but are not limited to, alleviation or amelioration of one or more cancer symptoms or conditions, diminishment or reduction of the extent of a cancer disease or of a cancer symptom, stabilizing, *i.e.,* not worsening, the state of a cancer disease or of a cancer symptom, prevention of a cancer disease or of a cancer symptom's spread, delay or slowing of cancer disease or cancer symptom progression, amelioration or palliation of the cancer disease state, diminishment of the reoccurrence of cancer disease, and remission, whether partial or total and whether detectable or undetectable. In other words, "**treatment**" as used herein includes any cure, amelioration, or reduction of a cancer disease or symptom. A **"reduction"** of a symptom or a disease means decreasing of the severity or frequency of the disease or symptom, or elimination of the disease or symptom.

As used herein, the terms **"therapeutically effective amount"** and **"prophylactically effective amount"** refer to an amount that provides a therapeutic benefit in the treatment, prevention, or management of pathological processes considered. The specific amount that is therapeutically effective can be readily determined by an ordinary medical practitioner and may vary depending on factors such as the type and stage of pathological processes considered, the patient's medical history and age, and the administration of other therapeutic agents.

The list of sources, ingredients, and components as described hereinafter are listed such that combinations and mixtures thereof are also contemplated and within the scope herein.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

All lists of items, such as, for example, lists of ingredients, are intended to and should be interpreted as Markush groups. Thus, all lists can be read and interpreted as items "selected from the group consisting of' ... list of items ... "and combinations and mixtures thereof."

Referenced herein may be trade names for components including various ingredients utilized in the present disclosure. The inventors herein do not intend to be limited by materials under any particular trade name. Equivalent materials (e.g., those obtained from a different source under a different name or reference number) to those referenced by trade name may be substituted and utilized in the descriptions herein.

### Compounds of the invention

The present invention is directed to bis-triazole 2,4,6-triarylpyridines compounds. More particularly, the compounds of the invention are of general formula (I): 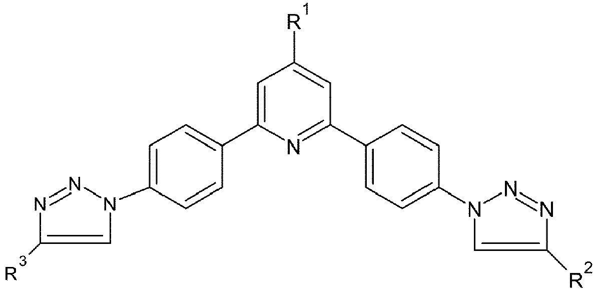 wherein
- R¹ may be a 5 to 6 membered unsaturated cycle, optionally comprising at least one heteroatom, and optionally substituted with either at least one:
   - C₁-C₄ saturated or unsaturated, linear or branched, alkyl group;
   - Y-R⁴ group with Y being N, O or S and R⁴ being either a C₁-C₄, saturated or unsaturated, linear or branched, alkyl group, or a benzyl group; or
   - halogen atom selected among F, Cl or Br;
- R² and R³, different or identical, in particular identical, are each -(CH₂)ₙ-Het, with
   - n ranging from 1 to 4, in particular from 2 to 3, and
   - Het being a saturated or unsaturated 5 to 6 membered heterocycle, optionally substituted with at least one C₁-C₄ saturated or unsaturated, linear or branched, alkyl group;
or a racemate, enantiomer, diastereoisomer of such compound or mixture thereof, or an addition salt of such compound, racemate, enantiomer, diastereomer or mixture with a mineral acid or organic acid.

R¹ may be 5 to 6 membered unsaturated cycle. A 5 to 6 membered unsaturated cycle may be an unsaturated cycloalkyl comprising carbon atoms or an unsaturated heterocycle comprising carbon atoms and at least one heteroatom.

A 5 to 6 membered unsaturated cycle may be partially or totally unsaturated, and in particular is totally unsaturated.

A 5 to 6 membered unsaturated cycloalkyl is in particular totally unsaturated or an aromatic cycle, and in particular is a phenyl.

A 5 to 6 membered unsaturated heterocycle may comprise as heteroatom O, N or S, and in particular comprises O or S. It may comprise one or two heteroatoms, and in particular it comprises one heteroatom. A 5 to 6 membered unsaturated heterocycle may be a thienyl, a furyl, a pyrrolyl, a pyrazolyl, an imidazolyl, a triazolyl, a tetrazolyl, an oxazolyl, a thiazolyl, an isoxazolyl, an isothiazolyl, a pyridinyl, a pyridazinyl, a pyrimidinyl, a pyrazinyl, a triazinyl, a pyranyl, a thiopyranyl, or an oxazinyl group.

In particular, a 5 to 6 membered unsaturated heterocycle may be a thienyl, a furyl, an imidazolyl, a pyrrolyl, or a pyrazolyl group.

In one embodiment R¹ may be a phenyl, a thienyl or a furyl.

The thienyl or furyl group may be bound to the pyridinyl group in position -2.

R¹ may optionally be substituted. In particular, when R¹ is an unsaturated heterocycle it is unsubstituted. In particular, when R¹ is an unsaturated cycloalkyl it is substituted. When R¹ is substituted, it may comprise at least one substituting group, in particular one, two or three substituting groups, and in particular only one substituting group.

R¹ may be substituted by at least one substituting group selected among:
- C₁-C₄ saturated or unsaturated, linear or branched, alkyl group;
- Y-R⁴ group with Y being N, O or S and R⁴ being either a C₁-C₄, saturated or unsaturated, linear or branched, alkyl group, or a benzyl group; or
- halogen atom selected among F, Cl or Br;

R¹ may be substituted by a C₁-C₄ alkyl group or by a Y-R⁴ group in which R⁴ may be either a C₁-C₄ alkyl group or a benzyl group.

A C₁-C₄ alkyl group may be saturated or unsaturated, linear or branched. In one embodiment, it may be a saturated, linear C₁-C₄ alkyl group. In another embodiment, it may be an unsaturated linear C₁-C₄ alkyl group. In another embodiment, it may be a saturated, branched C₁-C₄ alkyl group. In another embodiment, it may be an unsaturated, branched C₁-C₄ alkyl group. A substituting alkyl group may be a C₁-C₄, in particular it may be C₁-C₃ alkyl group, in particular a C₁-C₂ alkyl group, and in particular a C₁ alkyl group. It may be a methyl, an ethyl, a propyl, an isopropyl, a butyl, a sec-butyl, a *tert-*butyl.

In particular, when R¹ is substituted by a C₁-C₄ alkyl group, it may be substituted by a methyl or an ethyl group.

R¹ may be substituted by a Y-R⁴ group in which Y may be N, O or S, and R⁴ may be either a C₁-C₄ alkyl group as above defined or a benzyl group. In particular, Y is O or S. When R⁴ is a C₁-C₄ alkyl group, it may in particular be a methyl or an ethyl group, and in particular a methyl group.

In some embodiments, R¹ may be either an unsubstituted, unsaturated 5 to 6 membered cycle, comprising one O or S, or may be a phenyl group substituted with at least one group selected among Y-R⁴, Cl or Br, with Y being O or S and R⁴ being either a C₁-C₃, saturated or unsaturated, linear or branched, alkyl group or a benzyl group.

In another embodiment, R¹ may be selected among a furyl; a thienyl; or a phenyl substituted with at least one group selected among Y-R⁴, Cl or Br, wherein Y is O or S and R⁴ is either a C₁-C₂, saturated or unsaturated, alkyl group or a benzyl group.

In another embodiment, R¹ may be a phenyl substituted with at least one group being S-CH₃, O-benzyl, or Br, and in particular being S-CH₃.

In another embodiment, the phenyl is substituted with a single substituting group, in particular in position 4 of the phenyl.

In particular, when Y is O, then R⁴ is a benzyl group.

In particular, when Y is S, then R⁴ is a methyl or an ethyl group, and in particular is a methyl group.

R¹ may be substituted by a halogen atom selected among F, Cl or Br. In particular, R¹ may be substituted by Cl or by Br, and in particular it is substituted by Br.

In another embodiment, R¹ may be a phenyl substituted with at least one group being Y-R⁴ or Br, with Y being O or S and R⁴ being either a methyl or a benzyl group.

R¹ may be substituted with at least one of a C₁-C₄ alkyl group, Y-R⁴ group and/or a halogen atom as above defined. R¹ may be substituted with only one or several C₁-C₄ alkyl group. Alternatively, R¹ may be substituted with only one or several Y-R⁴ group. Alternatively, R¹ may be substituted with only one or several halogen group. Alternatively, R¹ may be substituted with a combination of at least one C₁-C₄ alkyl group, Y-R⁴ group and/or a halogen atom.

In particular, R¹ may be substituted with only one C₁-C₄ alkyl group. Alternatively, R¹ may be substituted with only one Y-R⁴ group. Alternatively, R¹ may be substituted with only one halogen atom.

In particular, when R¹ is a phenyl, the substituting group is in position -4 of the phenyl.

R² and R³ may be different or identical, and in particular are identical.

In some embodiments of the invention, in the compounds of the invention as above defined, R² and R³ are identical.

R² and R³ may be each -(CH₂)ₙ-Het, with:
- n ranging from 1 to 4, in particular from 2 to 3, and
- Het being a saturated or unsaturated 5 to 6 membered heterocycle group, optionally substituted with at least one C₁-C₄ saturated or unsaturated, linear or branched, alkyl group.

In some embodiments of the invention, n may be 2 or 3.

Het may be a saturated or unsaturated 5 to 6 membered heterocycle group. When unsaturated, it may be partially or totally unsaturated.

A 5 to 6 membered saturated or unsaturated heterocycle group may comprise as heteroatom O, N or S, and in particular comprises O or S. It may comprise one or two heteroatoms.

In some embodiments of the invention, Het may be a saturated 5 to 6 membered nitrogen heterocycle group comprising one or two nitrogen atoms.

A 5 to 6 membered saturated heterocycle group may be a pyrrolidinyl, a pyrazolidinyl group, an imidazolidinyl, a tetrahydrofuryl, a dioxolanyl, a tetrahydrothienyl, an oxathiolanyl, a piperidinyl, a piperazinyl, a tetrahydropyranyl, a dioxanyl, a thianyl, a dithianyl, a trithianyl, a morpholinyl, a thiomorpholinyl group.

A 5 to 6 membered unsaturated heterocycle group may be a thienyl, a furyl, a pyrrolyl, a pyrazolyl, an imidazolyl, a triazolylyl, a tetrazolyl, an oxazolyl, a thiazolyl, an isoxazolyl, an isothiazolyl, a pyridinyl, a pyridazinyl, a pyrimidinyl, a pyrazinyl, a triazinyl, a pyranyl, a thiopyranyl, or an oxazinyl group.

In particular, a 5 to 6 membered unsaturated heterocycle may be a thienyl, a furyl, an imidazolyl, a pyrrolyl, or a pyrazolyl group.

In particular, Het is a 5 to 6 membered saturated heterocycle group.

In particular, Het comprises one or two heteroatoms. In particular, the heteroatom is N.

In particular, Het is a pyrrolidinyl, a piperidinyl, or a piperazinyl group.

In particular, when Het comprises at least one N, it may be bound to -(CH₂)-via its, or one of its, N.

Het may be substituted or non-substituted. In particular, when Het is a pyrrolidinyl, a piperidinyl group it is a non-substituted. In particular, when Het is a piperazinyl group, it may be substituted, in particular in position -4.

Het may be substituted with at least one C₁-C₄ saturated or unsaturated, linear or branched, alkyl group. A C₁-C₄ alkyl group may be as above defined.

A C₁-C₄ alkyl group may be saturated or unsaturated, linear or branched. In one embodiment, it may a saturated, linear C₁-C₄ alkyl group. In another embodiment, it may be an unsaturated linear C₁-C₄ alkyl group. In another embodiment, it may be a saturated, branched C₁-C₄ alkyl group. In another embodiment, it may be an unsaturated, branched C₁-C₄ alkyl group. A substituting alkyl group may be a C₁-C₄, in particular it may be C₁-C₃ alkyl group, in particular a C₁-C₂ alkyl group, and in particular a C₁ alkyl group. It may be a methyl, an ethyl, a propyl, an isopropyl, a butyl, a sec-butyl, a *tert-*butyl.

In another embodiment, Het may be unsubstituted or may be substituted with at least one C₁-C₂, saturated or unsaturated, in particular unsaturated alkyl group, in particular with at least one methyl group.

In another embodiment, Het may be unsubstituted or may be substituted with one methyl group. In particular, Het may be substituted by a methyl group.

In another embodiment, Het may be either an unsubstituted pyrrolidine or piperidine or may be a piperazine substituted with a methyl group in position 4.

In particular, Het is a pyrrolidinyl, a piperidinyl, or a methyl-4 piperazinyl group.

According to some embodiments, the compounds of invention may be of general formula (I), wherein:
- R¹ may be a 5 to 6 membered unsaturated cycle substituted with either at least one:
   - Y-R⁴ group with Y being O or S and R⁴ being either a C₁-C₄ saturated or unsaturated, linear or branched, alkyl group or a benzyl group; or
   - halogen atom selected among F, Cl or Br;
- R² and R³, different or identical, in particular identical, are each -(CH₂)ₙ-Het, with
   - n ranging from 1 to 4, in particular from 2 to 3, and
   - Het being a saturated or unsaturated 5 to 6 membered heterocycle group, optionally substituted with a C₁-C₄ saturated or unsaturated, linear or branched, alkyl group.

In some embodiments, the compound of the invention may be of general formula (I), wherein:
- R¹ is a phenyl group substituted with either at least one:
   - Y-R⁴ group with Y being O or S and R⁴ being either a C₁-C₄ saturated, linear or branched, alkyl group or a benzyl group; or
   - halogen atom selected among F, Cl or Br;
- R² and R³, different or identical, in particular identical, are each -(CH₂)ₙ-Het, with
   - n ranging from 2 to 3, and
   - Het being a saturated 5 to 6 membered heterocycle group containing one or two N, optionally substituted with a C₁-C₄ saturated or unsaturated, linear or branched, alkyl group.

In some embodiments, the compound of the invention may be of general formula (I), wherein:
- R¹ is a phenyl group substituted with either at least one:
   - Y-R⁴ group with Y being O or S and R⁴ being either a C₁-C₃ saturated, linear or branched, alkyl group or a benzyl group; or
   - Cl or Br;
- R² and R³, different or identical, in particular identical, are each -(CH₂)ₙ-Het, with
   - n being 2 or 3, and
   - Het being a piperazinyl or a pyrrolidinyl group, optionally substituted with a C₁-C₃ saturated or unsaturated, linear or branched, alkyl group.

In some embodiments, the compound of the invention may be of general formula (I), wherein:
- R¹ is a phenyl group substituted with either at least one:
   - Y-R⁴ group with Y being O or S and R⁴ being either a C₁-C₂ saturated, alkyl group or a benzyl group; or
   - Cl or Br;
- R² and R³, different or identical, in particular identical, are each -(CH₂)ₙ-Het, with
   - n being 2 or 3, and
   - Het being a piperazinyl or a pyrrolidinyl group, optionally substituted with a C₁-C₂ saturated, alkyl group.

In some embodiments, the compound of the invention may be of general formula (I), wherein:
- R¹ is a phenyl group substituted with at least one S-R⁴ group with R⁴ being a methyl or an ethyl group, in particular a methyl group;
- R² and R³, identical, are each -(CH₂)ₙ-Het, with
- n ranging from 2 to 3, and
- Het being a piperazinyl or a pyrrolidinyl group, optionally substituted with a methyl or an ethyl group, in particular a methyl group.

In some embodiments, the compound of the invention may be of general formula (I), wherein:
- R¹ is a phenyl group substituted with at least one O-R⁴ group with R⁴ being a benzyl group;
- R² and R³, identical, are each -(CH₂)ₙ-Het, with
- n ranging from 2 to 3, and
- Het being a piperazinyl or a pyrrolidinyl group, optionally substituted with a methyl or an ethyl group, in particular a methyl group.

In some embodiments, the compound of the invention may be of general formula (I), wherein:
- R¹ is a phenyl group substituted with at least one Br;
- R² and R³, identical, are each -(CH₂)ₙ-Het, with
- n ranging from 2 to 3, and
- Het being a piperazinyl or a pyrrolidinyl group, optionally substituted with a methyl or an ethyl group, in particular a methyl group.

In some embodiments, a compound of the invention may be selected from the group consisting of formulas (IIa), (IIb), (IIc), (IId) or (IIe):

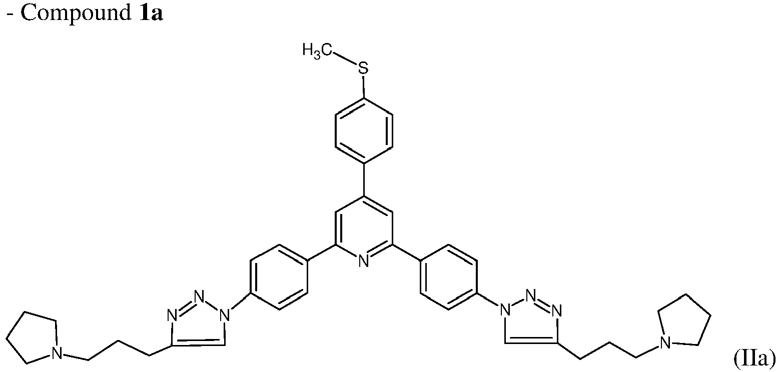

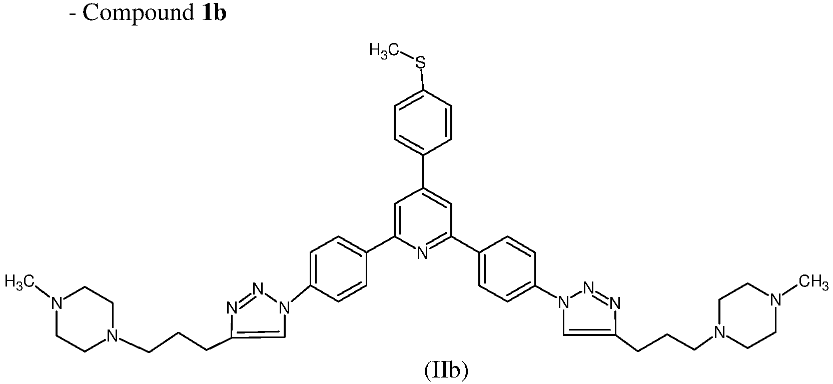

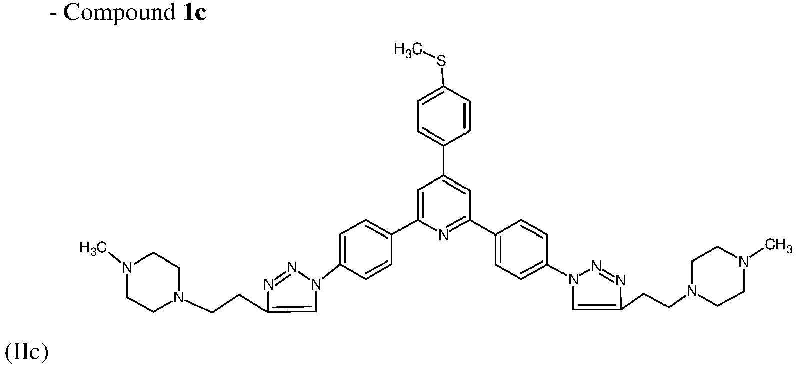

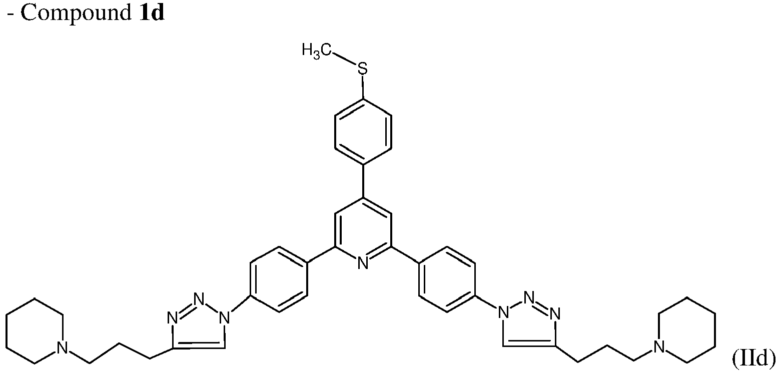

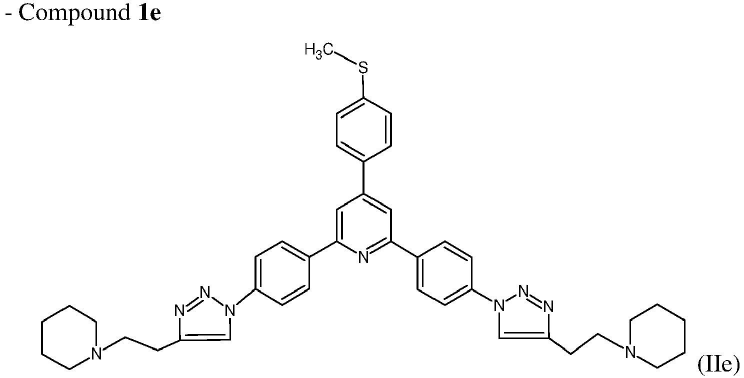

The compounds of the invention may be effective in the prevention and/or treatment of a cancer disease. Compounds of the invention may be used as a stand-alone therapeutic or prophylactic agent or also may be used in combination with another therapeutic agent, in particular with a lysosomotropic agent.

Alone or in combination with other therapeutic agents, the compounds of the invention are used at a therapeutically effective amount. The effective therapeutic amount may vary depending on several factors such as the nature and status of the cancer disease to be treated, the age, gender, weight of the patient, the concomitant presence of other diseases, the diet, the concomitant presence of other treatment. It is upon the skilled person to determine the appropriate therapeutically effective amount of a compound of the invention, depending on those factors and other well-known in the art.

A combination of a compound of the invention with a lysosomotropic agent, and in particular chloroquine or a derivative thereof, results in a synergistic effect. A synergistic effect of two agents, such as a compound of the invention and a lysosomotropic agent, in which the total effect of the combination is greater than the sum of the individual effect.

According to one embodiment, when used alone, a compound of the invention may be used in the range of 70 mg/day to 1400 mg/day. A skilled person will know how to adjust the concentration to use depending on the route of administration, the weight, age, gender of the patient to be treated, as well as depending on possible preexisting conditions to consider, and possible additional treatment administered.

According to another embodiment, when used in combination with another agent the combination of which resulting in a synergistic effect, such as a lysosomotropic agent, in particular such as chloroquine or a derivative thereof, a compound of the invention may be used in the range of 30 mg/day to 400 mg/day.

### Lysosomotropic agent

In some embodiments of the invention, a compound of the invention of general formula (I) as above defined may be used in combination with at least one lysosomotropic agent.

Lysosomotropic agent are compounds, lipophilic or amphiphilic with a basic moiety, able to be protonated and trapped within lysosomes (Giraldo et al., Biochem Soc Trans, 2014; Kuzu et al., Pharmacological Research, 2017).

Lysosomes are advanced organelles involved in many cellular processes and are considered crucial regulators of cell homoeostasis. The interior is acidic with a pH of < 5 and contains over 50 hydrolases, able to degrade all constituents of the cell. Lysosomes are limited by a single 7-10-nm phospholipid bilayer that functions as an interface to regulate communication between the lysosomal lumen and the cytosol. Owing to their high hydrolase content, leakage of lysosomal content to the cytosol is potentially harmful to the cell. Partial permeabilization of the membrane induces apoptosis, whereas massive lysosomal rupture induces necrosis. Lysosomal membrane permeabilization (LMP) is characterized as any damage to the lysosomal membrane that results in the release of the lysosomal contents into the cytosol

A lysosomotropic agent suitable for the invention is a compound able to relocate all or part of the lysosomal content to the cytosol. A lysosomotropic agent suitable for the invention is an active agent which accumulate inside acidic compartments.

As examples of methods suitable to identify lysosomotropic agent suitable for the invention, one may mention the imaging method described by Nadanaciva et al. (Toxicology in Vitro. 2011, 25(3), 715-723) or by Seo et al., (Biochemical and Biophysical Research Communications, 2014, 450(1), 189-194).

Among the lysosomotropic agents that may be useful for the invention, one may mention:
- chloroquine and derivatives thereof, such as oxychloroquine or hydroxychloroquine and chloroquine diphosphate. Chloroquine has already been proposed for the treatment of some cancer diseases;
- Lys05;
- DC661
- mefloquine
- siramesine;
- GNS561;
- nanaomycin;
- siomycin A;
- helenalin;
- or a pharmaceutically acceptable salt thereof.

**"Derivative"** has the definition as previously provided.

In embodiments, pharmaceutically acceptable salts of a lysosomotropic agent may also be used. A **"pharmaceutically acceptable salt**" has the definition as previously provided.

According to one embodiment, a lysosomotropic agent may be chloroquine; oxychloroquine; hydroxychloroquine; Lys05; siramesine; GNS561; or a pharmaceutically acceptable salt thereof.

According to one embodiment, a lysosomotropic agent may be chloroquine; oxychloroquine; hydroxychloroquine; or a pharmaceutically acceptable salt thereof.

According to one embodiment, a lysosomotropic agent may be chloroquine or a pharmaceutically acceptable salt thereof, and in particular is chloroquine diphosphate.

According to one embodiment, a lysosomotropic agent may be Lys05 or a pharmaceutically acceptable salt thereof, and in particular is Lys05.

According to one embodiment, a lysosomotropic agent may be selected among the group consisting in chloroquine; chloroquine diphosphate; hydroxychloroquine; and Lys05.

According to one embodiment, a lysosomotropic agent may be selected among the group consisting in chloroquine; Lys05; and a pharmaceutically acceptable salt thereof.

According to one embodiment, a lysosomotropic agent may be selected among the group consisting in chloroquine diphosphate and Lys05.

A lysosomal membrane permeabilization inducing agent is used, in combination with a compound of the invention, in a therapeutically effective amount. A **"therapeutically effective amount"** has the definition as previously provided. Dosages may be varied depending upon the requirements of the patient and the lysosomal membrane permeabilization inducing agents being employed.

A lysosomotropic agent, such as chloroquine or hydroxychloroquine, or a pharmaceutically acceptable thereof, may be administered at a therapeutically effective amount ranging from 10 to 1000 mg/dose, from 20 to 800 mg/dose, from 50 to 500 mg/dose, from 100 to 400 mg/dose.

For instance, a useful dosage for chloroquine may be in a range from 100 to 1000 mg/dose, from 150 to 800 mg/dose, or from 200 to 500 mg/dose.

### Combinations, Pharmaceutical compositions, and Kit-of-parts

According to some embodiments, the present invention is directed to a combination comprising at least one compound of the invention and at least one lysosomotropic agent.

It is to be understood that the term a **"combination"** as used herein envisages the simultaneous, sequential or separate administration of the components of the combination.

In one aspect of the invention, a combination envisages simultaneous administration of a compound of general formula (I) and of a lysosomotropic agent. In a further aspect of the invention, a combination envisages sequential administration of those agents. In another aspect of the invention, a combination envisages separate administration of those agents. Where the administration of those agents is sequential or separate, the delay in administering the second component should not be such as to lose the benefit of the effect of the combination therapy, in particular the synergistic effect.

The combination according to the invention comprises therapeutically active amount of the active agents. Accordingly, a combination in accordance with the invention is therapeutically active.

A combination according to the invention is able to induce lysosomal membrane permeabilization or LMP, and to trigger cancer cells death. The active agents of the combination of the invention, a compound of the invention and a lysosomotropic agent, are able to synergistically induce LMP and to synergistically trigger cancer cells death.

According to one embodiment, a compound of general formula (I) and a lysosomal membrane permeabilization inducing agent (lysosomotropic agent) may be each present in a combination according to the invention in a synergistic amount. A synergistic amount is, with respect to a given active agent, a fraction of the usually prescribed effective amount or therapeutically effective amount.

As used herein, an "**usually prescribed effective amount or therapeutically effective amount of an active agent**" refers to the amount or dose of a therapeutic agent typically used as a unit dose in the standard medical protocols or indicated in the accompanying documentation of the packages of the therapeutic agents known to the skilled person. This value, recognizable by the skilled person, varies from drug to drug and cannot be defined in a way applicable to all the commonly used therapeutic agents. The definition, applied to any active agent, indicates exactly the dosage commonly prescribed and specified in the medical and pharmacological protocols for therapy for each active agent known to the skilled person.

In one embodiment, a synergistically therapeutic efficient amount of a compound of general formula (I) may be about 0.1, 1.0, 2.0, 5.0, 10.0, 15.0, 20.0, 30.0, 40.0, 50.0, 70.0, 80.0, 90.0, 95.0, or 99.0% of the therapeutically efficient amount of the compound when used separately from the lysosomotropic agent.

In one embodiment, a synergistically therapeutic efficient amount of the lysosomotropic agent may be about 0.1, 1.0, 2.0, 5.0, 10.0, 15.0, 20.0, 30.0, 40.0, 50.0, 70.0, 80.0, 90.0, 95.0, or 99.0% of the therapeutically efficient amount of the lysosomotropic agent when used separately from the anticancer G4L.

The high content of hydrolytic enzymes in lysosomes makes them potentially harmful to the cell. If the lysosomal membrane is damaged (e.g. by membrane permeabilization), lysosomes release their contents into the cytosol, setting off indiscriminate degradation of cellular components. The distinctive sign of LMP is the translocation of soluble lysosomal components, such as cathepsins and other hydrolases, from the lysosomal lumen to the cytosol. Accordingly, LMP can be measured by a variety of simple techniques. Immunofluorescence techniques using antibodies against cathepsins such as CB and CD can reveal the redistribution of these proteases from lysosomes to the cytosol.

Several methods have been developed to detect LMP which can be useful to identify combinations suitable for the invention.

One may mention methods measuring the release into the cytosol of lysosomal enzymes such as cathepsins. These proteins can be visualized by fluorescence microscopy. Cathepsins confined within intact lysosomes are visualized as a punctate pattern of intense fluorescence and can be co-stained with antibodies against LAMP1 or LAMP2. By contrast, cathepsins released during LMP produce a diffuse fluorescence pattern throughout the cell. Cytosolic release of cathepsins can also be detected by Western blot of the cytosolic fractions after cell fractionation. This method can be used to estimate the extent of LMP by simultaneously determining the levels of several cathepsins of different sizes over time. Alternatively, cathepsins in cytosolic extracts can be detected using cathepsin-specific substrates (eg, Magic Red) and a fluorescence plate reader. Fluorescence microscopy can also be used to count cathepsin-positive cells in tissue sections. One can also use the Galactin puncta assay as described in Aits et al. (Sensitive detection of lysosomal membrane permeabilization by lysosomal galectin puncta assay. Autophagy. 2015; 11(8): 1408-1424. doi:10.1080/15548627.2015.1063871. Those methods, and others, are well-known to the skilled person.

According to another embodiment, the invention relates to a pharmaceutical composition comprising at least one compound of formula (I) and a pharmaceutically acceptable excipient or carrier.

According to another embodiment, the invention relates to a pharmaceutical composition comprising at least one combination comprising at least one compound of formula (I) and at least one lysosomotropic agent, and a pharmaceutically acceptable excipient or carrier.

Further, according to another embodiment, the invention is directed to a kit-of-parts comprising at least a first and a second containers, said first container containing at least a first composition comprising at least one compound of formula (I), and said second container containing at least a second composition comprising at least one lysosomotropic agent.

In some embodiments, the compositions of the kit-of-parts are pharmaceutical compositions.

In one embodiment, the lysosomotropic agent is as defined herein chloroquine, or a pharmaceutically acceptable salt thereof, such as chloroquine diphosphate, or is Lys05.

Compositions or combinations provided herein comprise the active agents in a therapeutically effective amount, *i.e.,* in an amount effective to achieve its intended purpose. As exposed above, the actual effective amount for a particular application will depend, *inter alia,* on the condition being treated and various other factors well-known in the art such as the age, the weight, the sex of the patient, the presence of other potential aggravating conditions, or the diet. Determination of a therapeutically effective amount of a compound of the invention is well within the capabilities of those skilled in the art.

The pharmaceutical compositions or combinations described herein can be prepared according to techniques known to the skilled person by using a compound of general formula (I), or combination of at least one compound of the invention and at least one lysosomotropic agent, in association with a pharmaceutically acceptable excipient or carrier.

These compositions may comprise one or more pharmaceutically acceptable excipients or carriers. Suitable carriers and excipients and their formulations are described, for example, in Remington: The Science and Practice of Pharmacy, 21st Edition, David B. Troy, ed., Lippicott Williams & Wilkins (2005). By pharmaceutically acceptable carrier is meant a material that is not biologically or otherwise undesirable, i.e., the material is administered to a subject without causing undesirable biological effects or interacting in a deleterious manner with the other components of the pharmaceutical composition in which it is contained.

The compositions can be in any form deemed appropriate by the skilled person, such as solid, semi-solid, liquid, granular, inhalation or aerosol inhalation.

The liquid forms may be appropriate forms for oral or systemic administration.

Compositions suitable for oral administration can be capsules, tablets, pills, powders, granules, solutions or suspensions in aqueous or non-aqueous liquids, foam or beaten edible, liquid oil in water emulsions or liquid water in oil emulsions.

For example, for oral administration in capsule or tablet form, the active agents mentioned herein may be combined with pharmaceutically acceptable inert carrier such as ethanol, glycerol, water and similar. There may also be present flavourings, preservative, colouring coating and/or dispersant agents.

Compositions suitable for parenteral administration may include sterile aqueous or non-aqueous solution for injection which may contain antioxidants, buffers, bacteriostatic and solutes which render the solution isotonic with the blood of the intended recipient, and aqueous or non-aqueous sterile suspensions which may include suspending and thickening agents. These compositions may be sterilized by conventional, well known sterilization techniques.

A parenteral composition may include a solution or suspension of the compounds in a vehicle such as sterile water or a parenterally acceptable oil. Alternatively, the solution can be lyophilized. The lyophilized parenteral pharmaceutical composition can be reconstituted with a suitable solvent just prior to administration.

The compositions may be presented in single dose or multi-dose containers, for example, sealed ampoules or vials, and may be stored in lyophilized condition requiring only the addition of the sterile liquid carrier, for example water for injections, immediately prior to use. Extemporaneous injection solutions and suspensions may be prepared from powders, granules, lyophilized and sterile compresses.

In the case of parenteral administration, the composition may also be provided with the active ingredients in separate containers that can be suitably admixed according to the desired dosage taking into account the weight, age, gender and health status of the patient in need thereof.

The compound of general formula (I) and the lysosomotropic agent may also be presented in separate compositions, packaged in separate containers, as a kit-of parts. The separate compositions may then be admixed before administration for a simultaneous administration, or they may be administered separately or sequentially.

### Cancer diseases, Therapeutic uses & Methods of treatment

In some embodiments, a compound of general formula (I), a combination or a pharmaceutical composition or a kit-of-parts according to the invention are for use in prevention and/or treatment of a cancer disease, in particular a chemoresistant cancer disease.

In some embodiments, the invention relates to the use of a compound of general formula (I) or of a combination according to the invention for the manufacture of a medicament for the prevention and/or treatment of a cancer disease, in particular a chemoresistant cancer disease.

In some embodiments, the invention relates to the use of a compound of general formula (I) or of a combination according to the invention for the manufacture of a kit-of-parts for the prevention and/or treatment of a cancer disease, in particular a chemoresistant cancer disease.

In other embodiments, the combination or the pharmaceutical composition or the kit-of-parts according to the invention are for use in synergistic prevention and/or treatment of a cancer disease.

In other embodiments, the combination or the pharmaceutical composition or the kit-of-parts according to the invention are for use in prevention and/or treatment of a chemoresistant cancer disease.

In other embodiments, the combination or the pharmaceutical composition or the kit-of-parts according to the invention are for use in a synergistic prevention and/or treatment of a chemoresistant cancer disease.

In some embodiments the manufactured medicament or kit-of-parts described herein are for a synergistic prevention and/or synergistic treatment of a cancer disease, in particular a chemoresistant cancer disease. As above indicated, "synergy" or "therapeutic synergy" are used when the combination of two products at given doses is more efficacious than the best of the two products alone considering the same doses.

Because of the synergistic effect of a combination according to the invention, each of the active agent, i.e., a compound of general formula (I) and a lysosomotropic agent, composing a combination of the invention may be present in an amount below their usual prescribed effective amount or therapeutically effective amount as active agent.

In other embodiments, a combination or a pharmaceutical composition or a kit-of-parts according to the invention comprises at least one compound of the invention in a synergistically therapeutic efficient amount and at least one lysosomotropic agent in a synergistically therapeutic efficient amount.

In some embodiments, the invention also relates to a method for preventing and/or treating a cancer disease in a subject in need thereof, said method includes administering to the subject a therapeutically effective amount of at least one compound of general formula (I), thereby treating a cancer disease in said subject.

In some embodiments, the invention also relates to a method for preventing and/or treating a cancer disease in a subject in need thereof, said method includes administering to the subject a therapeutically effective amount of at least one combination of the invention comprising at least one compound of general formula (I) and at least one lysosomotropic agent, thereby treating a cancer disease in said subject.

In some embodiments, the invention also relates to a method for preventing and/or treating a cancer disease in a subject in need thereof, said method includes administering to the subject a therapeutically effective amount of at least one pharmaceutical composition of the invention, thereby treating a cancer disease in said subject.

"**Patient**" or "**subject in need thereof"** as used herein refers to a living organism suffering from or prone to a disease or condition that can be treated by administration of a composition according to the invention. Non-limiting examples include mammals: human, bovines, rats, mice, dogs, monkeys, goat, sheep, cows, deer, or nonmammalian animals. In some embodiments, a subject or a patient is a mammal, and in particular is human.

In some embodiments, the invention also relates a method of synergistically preventing and/or treating a cancer disease in a subject in need thereof, said method includes administering to the subject a synergistically therapeutic effective amount of at least one compound of general formula (I) and at least one lysosomotropic agent, thereby treating a cancer disease in said subject. The methods of the invention include observing a prevention or a treatment, such as a relieving, of the cancer disease.

In some embodiments, the invention also relates to a method for the prevention and/or treatment of a chemoresistant and/or potentially chemoresistant cancer disease in a subject in need thereof, said method includes administering to the subject a therapeutically effective amount of at least one compound of general formula (I) alone, or in combination with at least one lysosomotropic agent, thereby treating a chemoresistant cancer disease in said subject. The methods of the invention include observing a prevention or a treatment, such as a relieving, of the chemoresistant and/or potentially chemoresistant cancer disease.

In some embodiments, the invention also relates to a method for the synergistic prevention and/or treatment of a chemoresistant and/or potentially chemoresistant cancer disease in a subject in need thereof, said method includes administering to the subject a synergistically therapeutic effective amount of at least one compound of the invention and at least one lysosomotropic agent, thereby treating a chemoresistant cancer disease in said subject. The methods of the invention include observing a synergistic prevention or a synergistic treatment, such as a relieving, of the cancer disease.

In some embodiments, in the methods described herein, a compound of the invention and a lysosomotropic agent are administered as a combination.

In some embodiments, in the methods described herein, a compound of the invention or a combination of the invention are administered as a pharmaceutical composition.

According to one embodiment, the invention is directed to a combination of the invention, or a pharmaceutical composition comprising such combination, or a kit-of-parts, or a method employing such combination, pharmaceutical composition, or kit-of-parts, as described here, wherein the compound of general formula (I) and the lysosomotropic agent are for simultaneous, separate, or sequential use.

A compound of the invention and a lysosomotropic agent may be administered in combination either simultaneously (*e.g.*, as a mixture), separately but simultaneously (e.g., via separate intravenous lines) or sequentially (*e.g*., one agent is administered first followed by administration of the second agent). Thus, the term combination is used to refer to concomitant, simultaneous or sequential administration of a compound of the invention and a lysosomotropic agent.

As used herein, **"administering"** or **"administered"** means administration by any route, such as oral administration, administration as a suppository, topical contact, parenteral, intraperitoneal, intramuscular, intralesional, intrathecal, intranasal, subcutaneous or transmucosal (*e.g.*, buccal, sublingual, palatal, gingival, nasal, vaginal, rectal, or transdermal) administration, or the implantation of a slow-release device, *e.g*., a mini-osmotic pump, to a subject, including parenteral. Parenteral administration includes intravenous, intramuscular, intra-arteriole, intradermal, subcutaneous, intraperitoneal, intraventricular, and intracranial.

Those terms are also meant to include modes of administration of the selected active agents, as considered in the present invention, to a single patient and are intended to include treatment regimens in which the agents are not necessarily administered by the same route of administration or at the same time. Administration can include delivery to a patient of the active agents, *e.g.* a compound of the invention and a lysosomotropic agent, simultaneously in the form of a single entity or dosage. Administration can also include that the active agents are both administered to a patient as separate entities either simultaneously, concurrently, or sequentially with no specific time limits, such that the administration provides therapeutically effective levels of the combination of active agents in the body of the patient.

In one embodiment, a compound of the invention and a lysosomotropic agent are administered simultaneously or separately.

In another embodiment, a compound of the invention and a lysosomotropic agent are administered sequentially.

In some embodiments, during the course of a treatment, a compound of the invention and a lysosomotropic agent may at times be administered sequentially and at other times be administered simultaneously or separately.

In some embodiments, where a compound of the invention and a lysosomotropic agent are administered sequentially, the compound of the invention is administered at a first time point and the lysosomotropic agent is administered at a second time point, wherein the first time point precedes the second time point. Alternatively, in embodiments, where the compound of the invention and the lysosomotropic agent are administered sequentially, the lysosomotropic agent is administered at a first time point and the anticancer G4L is administered at a second time point, wherein the first time point precedes the second time point.

In instances where the compound of the invention and the lysosomotropic agent are administered simultaneously, the active agents are admixed prior to administration.

In embodiments, uses or methods in accordance with the invention in particular implement compounds **1a, 1b, 1c**, **1d,** or **1e,** and in particular compounds **1a, 1b** or **1c**, and chloroquine, chloroquine diphosphate, or Lys05, as the lysosomotropic agent.

The course of a treatment with a combination of the invention is best determined on an individual basis, depending on the particular characteristics of the subject and the type of treatment selected. The treatment, such as those disclosed herein, can be administered to the subject on a daily, twice daily, bi-weekly, monthly or any applicable basis that is therapeutically effective. The treatment can be administered alone or in association with any other treatment disclosed herein or known in the art. The additional treatment can be administered simultaneously with the first treatment, at a different time, or on an entirely different therapeutic schedule (*e.g*., the first treatment can be daily, while the additional treatment is weekly).

In some embodiments, the combination or the pharmaceutical composition as described herein can be administered simultaneously, separately, or sequentially, daily for a period of time extending from at least one day up to several days, for instance up to 30 days, or several months, for instance up to 3 months. The administration may be repeated for several period of times separated by a period of time without administration. For instance, the administration may be carried out over two period of one month separated by a period of one month without administration. Such sequence may be repeated at least once or several times.

In some embodiments, the combination or the pharmaceutical composition as described herein can be administered once, twice, three time or 4 time a day, or once a day over a period of time ranging from a few minutes, *e.g*. one minutes, up to several minutes, *e.g.* 40, 50 or 60 minutes or up to several hours, one, two, three or up to 6 or 8 hours.

The exact dosage, frequency and length of administration will depend on the particular compound of general formula (I), or combination of compound of general formula (I) and lysosomotropic agent used, the particular condition to be treated, the severity of the condition to be treated, age, weight and the overall physical condition of the particular patient as well as on other medications that the patient is taking, as is well known to experts in the field. It is also clear that this quantity can be effectively lowered or increased depending on the responses of the treated patient and/or according to the evaluation of the physician prescribing the compounds of the present invention. The effective doses given here are therefore only indicative.

A cancer, in particular a chemoresistant cancer, which may be considered within the invention may be selected from breast cancer; colon cancer; rectal cancer; endometrial cancer; gastric carcinoma (including gastrointestinal carcinoid tumors and gastrointestinal stromal tumors); glioblastoma; hepatocellular carcinoma; cervical carcinoma; lung adeno-carcinoma (including small cell lung cancer and non-small cell lung cancer (NSCLC)); melanoma, including uveal melanoma; medulloblastoma; ovarian carcinoma; osteosarcoma; pancreatic cancer; prostate cancer; acute myelogenous leukemia (AML); chronic myelogenous leukemia (CML); non-Hodgkin's lymphoma; thyroid carcinoma; and pediatric tumors, such as leukemia, brain tumors, nephroblastoma, neuroblastoma, lymphoma, embryonic tumors, and rhabdosarcoma.

In a particular embodiment, the cancer disease considered within the invention is a cervical carcinoma, lung adeno-carcinoma, and osteosarcoma. The lung cancer may be in particular small cell lung cancer or non-small cell lung cancer (NSCLC).

The cancer, in particular the lung adeno-carcinoma, the cervical cancer or the osteosarcoma, may be a chemoresistant cancer.

Within the present description, the term "**chemoresistance**" or "**chemoresistant cancer**" refers to a reduction or a lack of responsivity of a cancer cell to a particular anticancer agent, used alone or in combination with other drugs commonly used in anticancer therapies. More particularly, a chemoresistant tumor or a chemoresistant cancer refers to a cancer that does not undergo cell death in response to a chemotherapeutic or anticancer agent.

As used herein "**compound, combination or composition effective in the treatment of chemoresistant and/or potentially chemoresistant cancer"** means compound, combination or composition which have the effect of inhibiting from the beginning the development of chemoresistant cells or to revert chemoresistance of cells treated with these substances and to restore the apoptotic pathway. A combination of the invention is a such composition.

It is to be understood that the invention encompasses all variations, combinations, and permutations in which one or more limitations, elements, clauses, descriptive terms, etc., from one or more of the listed claims which can be introduced into another claim dependent on the same base claim (or, as relevant, any other claim) unless otherwise indicated or unless it would be evident to one of ordinary skill in the art that a contradiction or inconsistency would arise. Where elements are presented as lists, (e.g., in Markush group or similar format) it is to be understood that each subgroup of the elements is also disclosed, and any element(s) can be removed from the group. It should be understood that, in general, where the invention, or aspects of the invention, is/are referred to as comprising particular elements, features, etc., certain embodiments of the invention or aspects of the invention consist, or consist essentially of, such elements, features, etc. For purposes of simplicity those embodiments have not in every case been specifically set forth in so many words herein. It should also be understood that any embodiment or aspect of the invention can be explicitly excluded from the claims, regardless of whether the specific exclusion is recited in the specification. The publications and other reference materials referenced herein to describe the background of the invention and to provide additional detail regarding its practice are hereby incorporated by reference.

The following examples are provided for purpose of illustration and not limitation.

### [EXAMPLES]

### Example 1: Materials and methods

### Cell culture

The human cervical cancer cell line HeLa and U2OS were purchased from the American Type Culture Collection (ATCC) and the human lung carcinoma A549 cell line is a generous gift of Prof. Jean-François Riou (CNRS UMR 7196, Paris France). The U2OS Galectin3-mCherry cell line was as described in Montespan et al. (PLoS Pathog 2017; 13: e1006217) or in Martinez et al. (Methods Mol Biol. 2013; 1064: 211-226). The lung adenocarcinoma PDX cell lines (PDX2) and (PDX3) are generous gifts from Dr. D. Santamaria (Ambrogio et al., Nat Med 2016; 22: 270-277).

HeLa cells were grown in RPMI 1640 culture media supplemented with 2 mM glutamine (Gibco-Life technologies), 10% fetal bovine serum (Gibco-Life technologies), 100 units/ml penicillin, and 100 µg/ml streptomycin (Gibco-Life technologies, 1540-122), while A549, U2OS and PDX cell lines were grown in DMEM, supplemented with 10% fetal bovine serum, 100 units/ml penicillin, and 100 µg/ml streptomycin (Gibco-Life technologies, 1540-122). For U2OS expressing Galectin3-mCherry 200 µg/ml hygromycin B (#10687010, Invitrogen) was added for cell culture, and removed from media before the experiments. All cell lines used in this study were cultivated at 37°C in a humidified atmosphere with 5% CO₂.

### Reagents

The Hoechst 33258 (#14530), Propidium iodide (#P4864) and Chloroquine diphosphate salt (#C6628) were purchased from Sigma-Aldrich.

### Compound 20A

The compound 20A was synthesized as described in Smith et al. (Org Biomol Chem. 2011 - compound n°3).

### Compounds 1a, 1b, 1c, 1d and 1e

The triarylpyridine compounds **1a, 1b, 1c**, **1d** and **1e** were synthesized as described in **Example 2.**

### Chemicals and instrumentation for chemical synthesis

Commercially reagents were used as received without additional purification. Melting points were determined with an SM-LUX-POL Leitz hot-stage microscope and are uncorrected. IR spectra were recorded on a NICOLET 380FT-IR spectrophotometer. NMR spectra were recorded with tetramethylsilane as an internal standard using a BRUKER AVANCE 300 spectrometer. Splitting patterns have been designated as follows: s = singlet; d = doublet; t = triplet; m = multiplet. Analytical TLC were carried out on 0.25 precoated silica gel plates (POLYGRAM SIL G/UV254) and visualization of compounds after UV light irradiation. Silica gel 60 (70-230 mesh) was used for column chromatography. High resolution mass spectra (electrospray in positive mode, ESI+ or MALDI-TOF MS) were recorded on a Waters Q-TOF Ultima apparatus. Elemental analyses were found within ±0.4% of the theoretical values.

### Quadruplex stabilization

Binding of compounds **1a, 1b** and **1c** to G-quadruplexes was investigated by FRET melting analysis as described in De Rache et al. (Biochimie 2015). Compounds **1a, 1b** and **1c** were tested at 1-10 µM concentration in the presence of the F21T oligonucleotide (FAM-5'-GGGTTAGGGTTAGGGTTAGGG-3'-TAMRA) synthesized by Eurogentec (Seraing, Belgium) and used without further purification at 0.2 µM strand concentration in a 10 mM lithium cacodylate pH 7.2 buffer supplemented with 10 mM KCl and 90 mM LiCl.

### Cell viability assay

MTT assay was used to monitor cell viability in HeLa cells subjected to different treatments. Briefly, cells were seeded in a 96-well plate (4 × 10³ cells/well). After 24h of treatment of cells with drug, MTT was added to each well to a final concentration of 0.5 mg/ml during 3h at 37 °C. The supernatant of cells was then removed, and 100 µl of DMSO was added per well. The absorbance in each well was measured at 570 nm and 630 nm using a Flexstation 3 microplate reader (Molecular Devices). 4 independent experiences were conducered, each performed in quadruplicate. To determine the IC₅₀ of compounds **20A**, **1a, 1b** and **1c**, a non-linear regression curve was fit using GraphPad Prism software.

### Lysosomal membrane permeabilization (LMP) analysis

### Quantification of Galectin 3 puncta

Lysosomal membrane damage was assessed thanks to U2OS expressing Galectin3-mCherry cells. Cells were plated on 12-well plates (7.5 × 10⁴ cells/well) treated with each compounds **20A**, **1a, 1b** and **1c** in either the presence or absence of 25 µM chloroquine for 24h. Cells were then washed with PBS, fixed with 4% (v/v) paraformaldehyde for 10 min at room temperature and then nucleic were counterstained for 15 min with Hoechst 33258. Image acquisition was made with a Leica DMI8 epifluorescence microscope, with a x20 objective (NA 0.40) equipped with a digital CMOS camera (Hamamatsu photonics) and filter bloc for detection of phase contrast and blue (exc: 325-375nm / em: 435-485nm) and red (exc: 541-551nm / em: 565-605nm) fluorescence. Each image was collected with identical exposure times and scaled equally. Five images were acquired for each condition and then analyzed thanks to ImageJ software (Schneider et al., 2012, 9(7): 671-675) in order to count manually the number of cells, and automatically the number of Galectin3 punctae within each cell by using the "Find Maxima" tool.

### Evaluation of cell death

Cell death was determined by measuring the plasma membrane permeability using propidium iodide dye (P4864, Sigma). Briefly, supernatant and attached cells were collected, pelleted at 1800 rpm for 5 min and loaded with 1 µg/ml propidium iodide for 15 min at room temperature. Cells were then analyzed by flow cytometry (FACSCalibur, Becton Dickinson) in the FL2 channel (exc: 488nm / em: 564-606nm).

### Statistical analysis.

Unless otherwise stated, each experiment was performed three times. Results were expressed as the mean value ± standard deviation (SD). Statistical analysis was performed by Mann-Withney unpaired statistical test. *p* < 0.05 was considered statistically significant. The software used was GraphPad PRISM 5.

### Example 2: Synthesis of bis-triazole triarylpyridines - compounds 1a, 1b 1c, 1d and 1e)

The compounds **1a, 1b, 1c**, **1d** and **1e** have been synthesized through a three steps procedure (**Figure 1**). The synthetic route involves the base-catalysed condensation of commercially available *p*-aminoacetophenone (***Sigma-Aldrich***) with 4-(methylthio)benzaldehyde (Sigma-Aldrich) in PEG300 resulting in the formation of the 2,6-bis(4-aminophenyl)-4-[4-(methylthio)phenyl]pyridine (**compound 2**) using the modified Chichibabin pyridine synthesis (Smith et al., Green Chemistry 2007; Zahmatkesh, Amino Acids 2011; Weiss, J. Am. Chem. Soc. 1952). This 2,6-bis(4-aminophenyl)pyridine (**compound 2**) was then converted into the intermediate corresponding diazido derivative (**compound 3**) which was then subjected to "click reaction", a copper-catalyzed azide-alkyne 1,3-dipolar cycloaddition reaction, with five amine-terminal alkynes to afford the substituted 1,2,3-triazole derivatives **1a, 1b, 1c**, **1d** and **1e.**

### Synthesis of compound 2 (2,6-bis(4-aminophenyl)-4-[4-(methylthio)phenyl] pyridine)

*p*-Aminoacetophenone (5 g, 37 mmol) was added to a suspension of crushed NaOH (1.5 g, 37 mmol) in PEG300 (20 mL) at room temperature. The suspension was stirred and heated to 80 °C to make a clear solution. Then, *p*-methylthiobenzaldehyde (2.8 g, 18.5 mmol) was added to the reaction mixture and heated at 110 °C for 4 h, after which NH₄OAc (42.7 g, 555 mmol) was added and the heating was reduced to 100 °C and stirred for 4 h. After cooling to room temperature, 400 mL cold water was added to the mixture to get a precipitate which was filtered and further purified through silica gel chromatography by using DCM:MeOH (0-5%) affording **compound 2.** Brown-yellow powder (52%); ¹H-NMR δ (300 MHz, CDCl₃) 8.37 (d, 4H, *J* = 8.70 Hz, 2H-2' and 2H-6'), 7.95 (s, 2H, H-3 and H-5), 7.92 (d, 4H, *J* = 8.70 Hz, 2H-3' and 2H-5'), 7.73 (d, 2H, *J* = 8.70 Hz, H-2" and H-6"), 7.44 (d, 2H, *J* = 8.70 Hz, H-3" and H-5"), 5.89 (br, 4H, 2NH₂), 2.59 (s, 3H, CH₃S). MALDI-TOF MS m/z [M + H]⁺ Calcd C₂₄H₂₁N₃S: 384.1529, Found: 384.1523. *(*Smith et al., Green Chemistry 2007*;* Zahmatkesh, Amino Acids 2011*;* Smith et al. Org. Biomol. Chem. 2011*)*

### Synthesis of compound 3 (2,6-bis(4-azidophenyl)-4-[4-(methylthio)phenyl] pyridine)

**Compound 2** (200 mg, 0.5 mmol) was added to 15 mL 6N HCl and the reaction mixture was cooled to 0 °C and NaNO₂ (138 mg, 2 mmol) was added and was stirred for 30 mins under cold condition. Sodium azide (195 mg, 3 mmol) was added to the reaction mixture portion-wise under cold condition; and the reaction mixture was allowed to stir at room temperature for 2 h. The mixture was neutralized with sodium bicarbonate and extracted with ethyl acetate (3x30 mL). The organic part was evaporated under vacuum at 30 °C to afford **compound 3.** Yellow crystals (98%); as a yellow solid which was used for next reaction. IR (KBr) 2105 cm-1 (N₃). MALDI-TOF MS m/z [M+H]⁺ Calcd for C₂₄H₁₈N₇S; 436.1339, Found: 436.1326.

### General procedure for the synthesis of 2,6-bis{4-[4-(substituted-aminoalkyl)-1H-1,2,3-triazol-1-yl]phenyl}-4-[4-(methylthio)phenyl]pyridines (Compounds 1a, 1b, 1c, 1d and 1e)

**Compound 3** (50 mg, 0.1 mmol) was added to a mixture of 4 mL H₂O and THF (1:1), then CuSO₄, 5H₂O (0.002 mmol) and sodium ascorbate (0.1 mmol) were added to the mixture and stirred. After 5 min, the substituted alkyne (0.3 mmol) was added and stirred for 12 h at room temperature. The solvent was evaporated under reduced pressure, and the residue was washed with water followed by diethyl ether to afford the desired bis-triazolylarylpyridine compounds **1a, 1b** and **1c**.

### Compound 1a (4-[4-(methylthio)phenyl]-2,6-bis{4-[4-(3-pyrrolidin-1-ylpropyl)-1H-1,2,3-triazol-1-yl)phenyl}pyridine)

Yellow crystals (84%); ¹H NMR δ (300 MHz, CDCl₃) 8.39 (d, 4H, *J* = 8.80 Hz, 2H-2' and 2H-6'), 7.96 (s, 2H, H-3 and H-5), 7.92 (d, 4H, *J* = 8.80 Hz, 2H-3' and 2H-5'), 7.86 (s, 2H, 2Htriazol.), 7.74 (d, 2H, *J* = 8.40 Hz, H-2" and H-6"), 7.43 (s, 2H, *J* = 8.40 Hz, H-3" and H-5"), 2.91 (t, 4H, *J* = 7.50 Hz, 2NCH₂), 2.63-2.53 (m, 12H, 6NCH₂), 2.59 (s, 3H, CH₃S), 2.06-1.96 (m, 4H, 2CH₂pyrrol.), 1.84-1.77 (m, 4H, 2CH₂pyrrol.). ¹³C NMR δ (75 MHz, CDCl₃) 157.5 (C-2 and C-6), 151.4 (C-4), 150.2 (C-4triazol.), 142.1 (C-4"), 140.6 (C-1'), 138.9 (C-4'), 135.9 (C-1"), 129.8 (C-3' and C-5'), 128.7 (C-2" and C-6"), 127.9 (C-3" and C-5"), 121.8 (C-2' and C-6'), 120.2 (C-5triazol.), 118.5 (C-3 and C-5), 58.3 (NCH₂), 56.8 (NCH₂), 28.4 (CH₂), 27.4 (CH₂), 24.9 (CH₂), 16.8 (CH₃S). MALDI-TOF MS m/z [M+H]⁺ Calcd for C₄₂H₄₈N₉S; 710.3748, Found: 710.3740.

### Compound 1b (2,6-bis{4-[4-(3-(4-methylpiperazin-1-yl)propyl)-1H-1,2,3-triazol-1-yl]phenyl}-4-[4-(methylthio)phenyl]pyridine)

Orange crystals (68 %); 1H NMR δ (300 MHz, CDCl₃) 8.38 (d, 4H, *J* = 8.70 Hz, 2H-2' and 2H-6'), 7.96 (s, 2H, H-3 and H-5), 7.92 (d, 4H, *J* = 8.70 Hz, 2H-3' and 2H-5'), 7.86 (s, 2H, 2Htriazol.), 7.73 (d, 2H, *J* = 8.50 Hz, H-2" and H-6"), 7.43 (d, 2H, *J* = 8.50 Hz, H-3" and H-5"), 2.89 (t, 4H, *J* = 7.50 Hz, 2NCH₂), 2.66-2.42 (m, 20 H, 2NCH₂ and 8NCH₂piperaz.), 2.59 (s, 3H, CH₃S), 2.32 (s, 6H, 2NCH₃), 2.02-1.92 (m, 4H, 2CH₂). ¹³C NMR δ (75 MHz, CDCl₃) 157.5 (C-2 and C-6), 151.3 (C-4), 150.1 (C-4triazol.), 142.1 (C-4"), 140.6 (C-1'), 139.0 (C-4'), 136.1 (C-1"), 129.7 (C-3' and C-5'), 128.8 (C-2" and C-6"), 128.0 (C-3" and C-5"), 121.7 (C-2' and C-6'), 120.2 (C-5triazol.), 118.4 (C-3 and C-5), 59.1 (NCH₂), 56.5 (NCH₂), 54.5 (NCH₂), 47.4 (NCH₃), 28.0 (CH₂), 25.0 (CH₂), 16.8 (CH₃S). MALDI-TOF MS m/z [M+H]⁺ Calcd for C₄₄H₅₄N₁₁S; 768.4279, Found: 768.4269.

### Compound 1c (2,6-bis{4-[4-(2-(4-methylpiperazin-1-yl)ethyl)-1H-1,2,3-triazol-1-yl]phenyl}-4-[4-(methylthio)phenyl]pyridine)

Yellow crystals (64%); ¹H NMR δ (300 MHz, CDCl₃) 8.39 (d, 4H, *J* = 8.80 Hz, 2H-2' and 2H-6'), 7.96 (s, 2H, H-3 and H-5), 7.94 (s, 2H, 2Htriazol.), 7.91 (d, 4H, *J* = 8.80 Hz, 2H-3' and 2H-5'), 7.73 (d, 2H, *J* = 8.40 Hz, H-2" and H-6"), 7.43 (d, 2H, *J* = 8.40 Hz, H-3" and H-5"), 3.07 (t, 4H, *J* = 7.80 Hz, 2NCH₂), 2.81 (t, 4H, *J* = 7.80 Hz, 2NCH₂), 2.63-2.43 (m, 16 H, 8NCH₂piperaz.), 2.59 (s, 3H, CH₃S), 2.34 (s, 6H, 2NCH₃), ¹³C NMR δ (75 MHz, CDCl₃) 157.5 (C-2 and C-6), 151.5 (C-4), 148.0 (C-4triazol.), 142.2 (C-4"), 140.8 (C-1'), 139.0 (C-4'), 136.2 (C-1"), 129.8 (C-3' and C-5'), 128.8 (C-2" and C-6"), 128.0 (C-3" and C-5"), 121.9 (C-2' and C-6'), 120.8 (C-5triazol.), 118.5 (C-3 and C-5), 58.6 (NCH₂), 55.8 (NCH₂), 53.1 (NCH₂), 46.5 (NCH₃), 24.6 (CH₂), 16.8 (CH₃S). MALDI-TOF MS m/z [M+H]⁺ Calcd for C₄₂H₅₀N₁₁S; 740.3966, Found: 740.3924.

### Compound 1d (2,6-bis{4-[4-(3-piperidin-1-ylpropyl)-1H-1,2,3-triazol-1-yl]phenyl}-4-[4-(methylthio)phenyl]pyridine)

Yellow crystals(73 %); ¹H NMR δ (300 MHz, CDCl₃) 8.36 (d, 4H, *J* = 8.70 Hz, 2H-2' and 2H-6'), 7.95 (s, 2H, H-3 and H-5), 7.91(d, 4H, *J* = 8.70 Hz, 2H-3' and 2H-5'), 7.85 (s, 2H, 2Htriazol.), 7.72 (d, 2H, *J* = 8.50 Hz, H-2" and H-6"), 7.42 (s, 2H, *J* = 8.50 Hz, H-3" and H-5"), 2.87 (t, 4H, *J* = 7.50 Hz, 2NCH₂), 2.59 (s, 3H, CH₃S), 2.47-2.40 (m, 12H, 6NCH₂), 1.99 (qt, 4H, *J* = 7.50 Hz, 2CH₂), 1.61 (qt, 8H, *J* = 5.40 Hz, 4CH₂piper.), 1.50-1.43 (m, 4H, 2CH₂piper.). ¹³C NMR δ (75 MHz, CDCl₃) 157.6 (C-2 and C-6), 151.4 (C-4), 150.3 (C-4triazol.), 141.0 (C-4"), 140.7 (C-1'), 139.1 (C-4'), 136.2 (C-1"), 129.8 (C-3' and C-5'), 128.8 (C-2" and C-6"), 128.1 (C-3" and C-5"), 121.8 (C-2' and C-6'), 120.2 (C-5triazol.), 118.4 (C-3 and C-5), 60.1 (NCH₂), 56.0 (NCH₂), 28.1 (CH₂), 27.4 (CH₂), 25.8 (CH₂), 25.1 (CH₂), 16.8 (CH₃S). MALDI-TOF MS m/z [M+H]⁺ Calcd for C₄₄H₅₂N₉S: 738.4061; Found: 738.4053.

### Compound 1e (2,6-bis{4-[4-(2-piperidin-1-ylethyl)-1H-1,2,3-triazol-1-yl]phenyl}-4-[4-(methylthio)phenyl]pyridine)

Yellow crystals (77 %); ¹H NMR δ (300 MHz, CDCl₃) 8.35 (d, 4H, *J* = 8.70 Hz, 2H-2' and 2H-6'), 7.94 (s, 2H, H-3 and H-5), 7.92(d, 4H, *J* = 8.70 Hz, 2H-3' and 2H-5'), 7.75 (s, 2H, 2Htriazol.), 7.74(d, 2H, *J* = 8.50 Hz, H-2" and H-6"), 7.44 (d, 2H, *J* = 8.50 Hz, H-3" and H-5"), 3.72 (t, 4H, *J* = 6.60 Hz, 2NCH₂), 2.70 (t, 4H, *J* = 6.60 Hz, 2NCH₂), 2.58 (s, 3H, CH₃S), 2.55-2.45 (m, 8H, 4NCH₂), 1.64-1.61 (m, 8H, 4CH₂piper.),1.49-1.42 (m, 4H, 2CH₂piper.). ¹³C NMR δ (75 MHz, CDCl₃) 157.5 (C-2 and C-6), 151.3 (C-4), 150.2 (C-4triazol.), 140.9 (C-4"), 140.6 (C-1'), 139.0 (C-4'), 136.1 (C-1"), 129.7 (C-3' and C-5'), 128.8 (C-2" and C-6"), 128.0 (C-3" and C-5"), 121.7 (C-2' and C-6'), 120.2 (C-5triazol.), 118.3 (C-3 and C-5), 58.9 (NCH₂), 55.8 (NCH₂), 26.8 (CH₂), 25.2 (CH₂), 24.9 (CH₂), 16.8 (CH₃S). MALDI-TOF MS m/z [M+H]⁺ Calcd for C₄₂H₄₈N₉S; 710.3748, Found: 710.3743.

### Example 3: Binding to G-quadruplex structure

The new triarylpyridine compounds, **1a, 1b,** and **1c** were assayed for capacity to recognize and stabilize G-quadruplex (G4). FRET melting experiments were conducted using the F21T oligonucleotide.

It has been observed that an increasing concentration of each compound (1-10 µM) led to an increase in melting temperature (Tm). Therefore, confirmation of G4 recognition was obtained and all three compounds stabilize the human telomeric quadruplex to comparable extents (**Figure 2**).

### Example 4: Compounds 1a, 1b and 1c have better IC50 than 20A in inducing Hela cells death

The IC₅₀ in HeLa cells was determined for the three compounds (1a, 1a and 1c). As shown in **Figure 3**, these compounds significantly inhibited HeLa cell viability. The IC₅₀ values of the compounds were calculated as a mean from 4 independent experiments performed in quadruplicate. The IC₅₀ are, respectively, **1a:** 4.34 µM, **1b:** 3.15 µM, and **1c:** 3.62 µM, which are even lower than the IC₅₀ of **20A:** 6.35 µM.

A sublethal doses (mortality < 10%, as for 20A) was then selected for each compound to analyze lysosomal membrane permeabilization (LMP) and cell death in the presence or absence of chloroquine.

### Example 5: Compounds 1a, 1b and 1c activate LMP and chloroquine significantly increases LMP triggered by compounds 1a, 1b and 1c

LMP was analysed by counting Galectin-3 positive cells. As shown in **Figure 4**, unlike compound **20A**, all three compounds (**1a-1c**) have the ability to induce LMP alone (compared to untreated cells - DMSO) as evidenced by the percentage of cells with Gal3 puncta which corresponds to 26% (**#1a**), 31% (**#1b**) and 25% (**#1c**). More importantly, the percentage of Gal3 puncta positive cells was further increased when chloroquine was added to each ligand (57%, 56% and 45% for **1a, 1b** and **1c**, respectively, **Figure 4**). This response was accompanied by an increase in the percentage of cells harboring more than three Gal3 punctae (**Figure 5**), suggesting that a significant population of cells had undergone intensive lysosomal membrane damage. Thus, the combination of chloroquine and compounds **1a, 1b** and **1c** triggered a massive increase in LMP induction.

Those results clearly showed that new compounds **1a, 1b** and **1c** caused an important damage to the lysosomal membrane and perform much better than the **20A** compound with and without chloroquine.

Further, these results show that the new triarylpyridine compounds, **1a, 1b** and **1c**, can act as lysosomal membrane permeabilization inducing agents.

### Example 6: Combined treatment with chloroquine and compounds 1a, 1b, 1c significantly activates cell death in lung adenocarcinoma cells

It was investigated whether the combined treatment with chloroquine and compounds **1a, 1b,** and **1c** could exacerbate cell death modalities. The induction of cell death in response to compounds **20A**, **1a, 1b** and **1c** in either the presence or absence of chloroquine in A549 lung adenocarcinoma cells was compared.

As shown in **Figure 6**, cell death was markedly enhanced when chloroquine (CQ) was combined with **1a** (80% of cell death vs. 13% of cell death without CQ). Similar results are also observed with **1b** (40 % of cell death vs. 2% of cell death without CQ) and **1c** (70% of cell death vs. 5% of cell death without CQ), supporting the idea that the trialypyridine compounds **1a, 1b** and **1c** cooperate with chloroquine to trigger a robust cell death.

Further, compounds **1a, 1b** and **1c** show an improved capacity to induce cell death in cancer cells, alone or in combination with a lysosomotropic agent such as chloroquine, in comparison with compound **20A.**

To validate the results in a clinically relevant setting, it was also examined the effect of combination of trialpyridine compounds **1a, 1b** and **1c** with chloroquine in patient-derived xenograft cell lines from lung adenocarcinomas (referred as PDX2 and PDX3 in the original paper Ambrogio et al.(Nat. Med. 2016, 22, 270-277). As shown in **Figure 7**, both PDX cell lines were highly sensitive to the combined treatment. For PDX2 cells with **1a** (59% of cell death vs. 10% of cell death without CQ), **1b** (30 % of cell death vs. 8% of cell death without CQ) and **1c** (60% of cell death vs. 10% of cell death without CQ) (**Figure 7A**). Similar results are also observed for PDX3 cells with **1a** (59% of cell death vs. 8% of cell death without CQ), **1b** (25 % of cell death vs. 5% of cell death without CQ) and **1c** (55% of cell death vs. 8% of cell death without CQ) (**Figure 7B**), suggesting the relevance of this combination for further preclinical studies in lung cancer models.

Those results show and confirm that combination of a lysosomotropic agent, such as chloroquine, with the new triarylpyridine compounds, **1a, 1b** or **1c**, acted synergistically to induce cell death.

In conclusion, altogether, these results showed that the new triarylpyridine compounds of the invention, illustrated by compounds **1a, 1b** and **1c** induce cancer cell deaths alone or in combination with a lysosomotropic agent, such as chloroquine. The combination of the new triarylpyridine compounds of the invention with a lysosomotropic agent further advantageously induce a synergistic effect on the induction of cancer cells death.

The results here show the usefulness of the new triarylpyridine compounds of the invention, alone or in combination with a lysosomotropic agent, for preventing and/or treating a cancer disease.

### [REFERENCES]

Aits S, Kricker J, Liu B, et al. Sensitive detection of lysosomal membrane permeabilization by lysosomal galectin puncta assay. Autophagy. 2015;11(8):1408-1424. doi: 10.1080/15548627.2015.1063871
Appelqvist, H.; Wäster, P.; Kågedal, K.; Ollinger, K. The lysosome: from waste bag to potential therapeutic target. J Mol Cell Biol 2013, 5, 214-226, doi: 10.1093/jmcb/mjt022.
Ambrogio, C.; Gómez-López, G.; Falcone, M.; Vidal, A.; Nadal, E.; Crosetto, N.; Blasco, R.B.; Fernández-Marcos, P.J.; Sánchez-Céspedes, M.; Ren, X.; et al. Combined inhibition of DDR1 and Notch signaling is a therapeutic strategy for KRAS-driven lung adenocarcinoma. Nat. Med. 2016, 22, 270-277, doi: 10. 103 8/nm.404 1.
Asamitsu S, Obata S, Yu Z, Bando T, and Sugiyama H. Recent Progress of Targeted G-Quadruplex-Preferred Ligands Toward Cancer Therapy. Molecules 2019; 254:429.
De Rache, A.; Mergny, J.-L. Assessment of selectivity of G-quadruplex ligands via an optimised FRET melting assay. Biochimie 2015, 115, 194-202, doi:10.1016/j.biochi.2015.06.002.
Giraldo A.V.M., Appelqvist H., Ederth T. and Ollinger K., Lysosomotropic agents: impact on lysosomal membrane permeabilization and cell death. Biochem. Soc. Trans. 2014; 42, 1460-1464
Gong, Y.; Duvvuri, M.; Duncan, M.B.; Liu, J.; Krise, J.P. Niemann-Pick C1 protein facilitates the efflux of the anticancer drug daunorubicin from cells according to a novel vesicle-mediated pathway. J. Pharmacol. Exp. Ther. 2006, 316, 242-247, doi: 10.1124/jpet. 105.089482.
Herlevsen, M.; Oxford, G.; Owens, C.R.; Conaway, M.; Theodorescu, D. Depletion of major vault protein increases doxorubicin sensitivity and nuclear accumulation and disrupts its sequestration in lysosomes. Mol. Cancer Ther. 2007, 6, 1804-1813, doi:10.1158/1535-7163.MCT-06-0372.
Kroemer, G.; Jäättelä, M. Lysosomes and autophagy in cell death control. Nature Reviews Cancer 2005, 5, 886-897, doi:10.1038/nrcl738.
Kroemer G and Galluzzi L. Lysosome-targeting agents in cancer therapy. Oncotarget 2017; 8: 112168-112169.
Kuzu O.F., Toprak M. Noory A.M. and Robertson G.P., Effect of lysosomotropic molecules on cellular homeostasis. Pharmacological Research, 2017, 117, 177-184
Li Q, Xiang JF, Zhang H and Tang YL. Searching Drug-Like Anti-cancer Compound(s) Based on G-Quadruplex Ligands. Current Pharmaceutical Design 2012; 18, 1973-1983.
Martinez R., Burrage A.M., Wiethoff C.M., and Wodrich H.. High temporal resolution imaging reveals endosomal membrane penetration and escape of adenoviruses in real-time. Methods Mol Biol. 2013; 1064: 211-226.
Montespan, C.; Marvin, S.A.; Austin, S.; Burrage, A.M.; Roger, B.; Rayne, F.; Faure, M.; Campell, E.M.; Schneider, C.; Reimer, R.; et al. Multi-layered control of Galectin-8 mediated autophagy during adenovirus cell entry through a conserved PPxY motif in the viral capsid. PLoS Pathog. 2017, 13, e1006217, doi: 10. 137 1/joumal.ppat. 1006217.
Nadanaciva S., Lu S., Gebhard D.F., Jessen B.A., Pennie W.D. and Will Y. A high content screening assay for identifying lysosomotropic compounds. Toxicology in Vitro. 2011, (25)3, 715-723
Schneider, C. A.; Rasband, W. S. & Eliceiri, K. W. "NIH Image to ImageJ: 25 years of image analysis", Nature methods, 2012, 9(7): 671-675.
Seo I., Jha B.K., Lim JG., Suh SI., Suh MH., and Baek WK., Identification of lysosomotropic compounds based on the distribution and size of lysosomes, Biochemical and Biophysical Research Communications, 2014, 450(1), 189-194
Smith, N.M.; Labrunie, G.; Corry, B.; Tran, P.L.T.; Norret, M.; Djavaheri-Mergny, M.; Raston, C.L.; Mergny, J.-L. Unraveling the relationship between structure and stabilization of triarylpyridines as G-quadruplex binding ligands. Org. Biomol. Chem. 2011, 9, 6154-6162, doi:10.1039/c1ob05560g.
Smith, N.M.; Raston, C.; Smith, C.B.; Sobolev, A.N. PEG mediated synthesis of amino-functionalised 2,4,6-triarylpyridines. Green Chemistry 2007, 9, 1185-1190, doi:10.1039/b700893g.
Weiss, M. Acetic Acid-Ammonium Acetate Reactions. An Improved Chichibabin Pyridine Synthesis 1. J. Am. Chem. Soc. 1952, 74, 200-202, doi:10.1021/ja01121a051.
Xu, H.; Ren, D. Lysosomal physiology. Annu. Rev. Physiol. 2015, 77, 57-80, doi: 10. 1 146/annurev-physiol-021014-071649.
Zahmatkesh, S. Ionic liquid catalyzed synthesis and characterization of heterocyclic and optically active poly (amide-imide)s incorporating L-amino acids. Amino Acids 2011, 40, 533-542, doi:10.1007/s00726-010-0667-3.
Zhitomirsky, B.; Assaraf, Y.G. Lysosomal sequestration of hydrophobic weak base chemotherapeutics triggers lysosomal biogenesis and lysosome-dependent cancer multidrug resistance. Oncotarget 2015, 6, 1143-1156, doi:10.18632/oncotarget.2732.

## Claims

1. A compound of general formula (I): 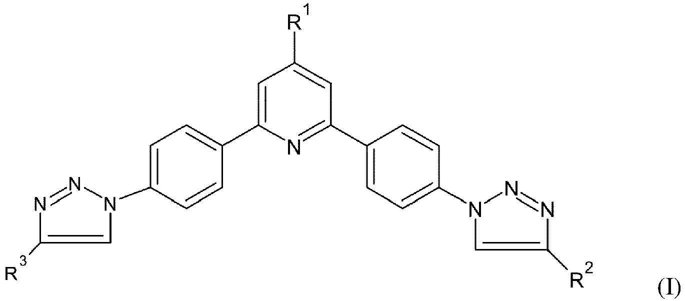 wherein
- R¹ is a 5 to 6 membered unsaturated cycle, optionally comprising at least one heteroatom, and optionally substituted with either at least one:
- C₁-C₄ saturated or unsaturated, linear or branched, alkyl group;
- Y-R⁴ group with Y being N, O or S and R⁴ being either a C₁-C₄, saturated or unsaturated, linear or branched, alkyl group, or a benzyl group; or
- halogen atom selected among F, Cl or Br;
- R² and R³, different or identical, in particular identical, are each -(CH₂)ₙ-Het, with
- n ranging from 1 to 4, in particular from 2 to 3, and
- Het being a saturated or unsaturated 5 to 6 membered heterocycle group, optionally substituted with at least one C₁-C₄ saturated or unsaturated, linear or branched, alkyl group;
or a racemate, enantiomer, diastereoisomer of such compound or mixture thereof, or an addition salt of such compound, racemate, enantiomer, diastereomer or mixture with a mineral acid or organic acid.

2. The compound according to claim 1, wherein:
- R¹ is a 5 to 6 membered unsaturated cycle, optionally comprising at least one O or S, and optionally substituted with at least one group being:
- Y-R⁴ with Y being O or S and R⁴ being either a C₁-C₃, saturated or unsaturated, linear or branched, alkyl group or a benzyl group; or
- Cl or Br;
- R² and R³, different or identical, in particular identical, are each -(CH₂)ₙ-Het, with
- n being 1, 2 or 3, in particular 2 or 3, and
- Het being a saturated or unsaturated 5 to 6 membered nitrogen heterocycle group, optionally substituted with at least one C₁-C₃ saturated or unsaturated, linear or branched, alkyl group.

3. The compound according to anyone of claim 1 or 2, wherein R¹ is either an unsubstituted, unsaturated 5 to 6 membered cycle, comprising one O or S, or is a phenyl group substituted with at least one group selected among Y-R⁴, Cl or Br, with Y being O or S and R⁴ being either a C₁-C₃, saturated or unsaturated, linear or branched, alkyl group or a benzyl group, and in particular R¹ is selected among a furyl; a thienyl; or a phenyl substituted with at least one group selected among Y-R⁴, Cl or Br, wherein Y is O or S and R⁴ is either a C₁-C₂, saturated or unsaturated, alkyl group or a benzyl group.

4. The compound according to anyone of the preceding claims, wherein R¹ is a phenyl substituted with at least one group, in particular is substituted with a single substituting group, in particular in position 4 of the phenyl, said group being Y-R⁴ or Br, with Y being O or S and R⁴ being either a methyl or a benzyl group.

5. The compound according to anyone of preceding claims, wherein R¹ is a phenyl substituted with at least one group, in particular is substituted with a single substituting group, in particular in position 4 of the phenyl, said group being S-CH₃, O-benzyl, or Br, and in particular is S-CH₃.

6. The compound according to anyone of the preceding claims, wherein Het is a saturated 5 to 6 membered nitrogen heterocycle group comprising one or two nitrogen atoms, in particular Het is selected among a pyrrolidinyl, a piperidinyl, or a piperazinyl group, in particular Het is unsubstituted or is substituted with at least one C₁-C₂, saturated or unsaturated, alkyl group, and in particular Het is substituted with at least one methyl group.

7. The compound according to anyone of the preceding claims, wherein Het is either an unsubstituted pyrrolidine or piperidine or is a piperazine substituted with a methyl group in position 4.

8. The compound according to anyone of preceding claims, which is selected from the group consisting of formulas (IIa), (IIb), (IIc), (IId) or (IIe):

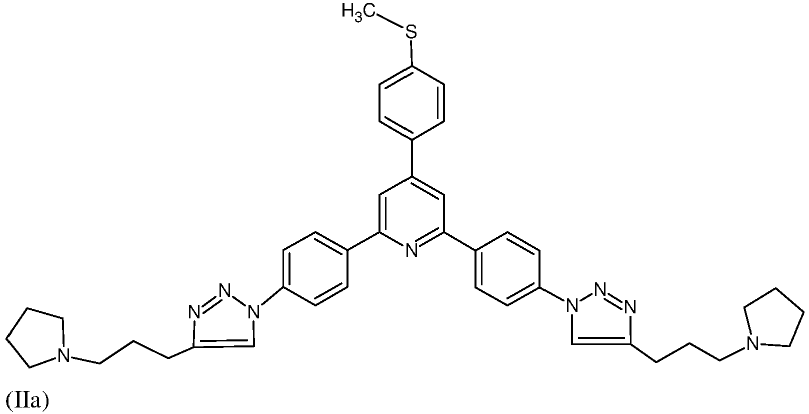

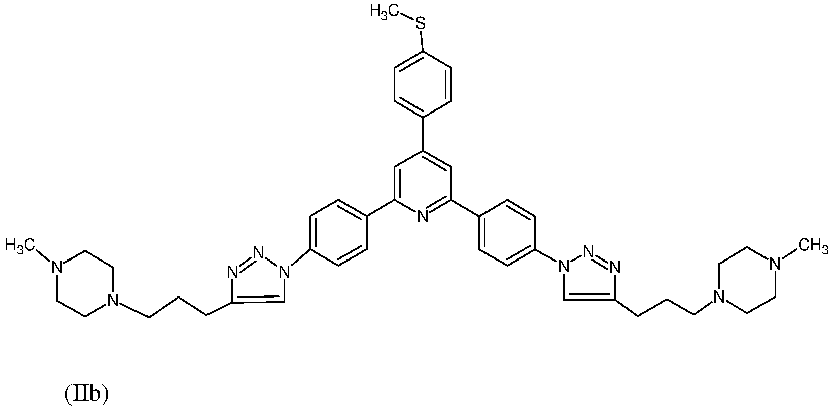

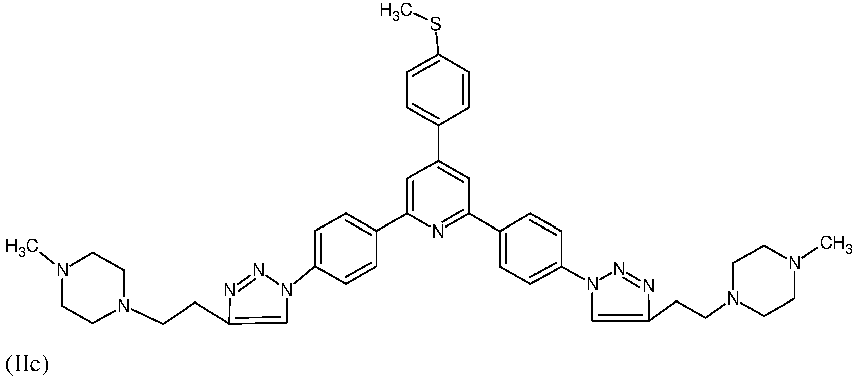

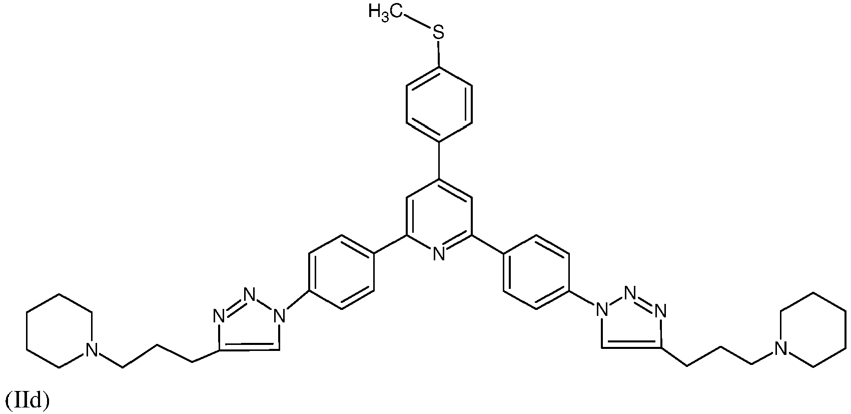

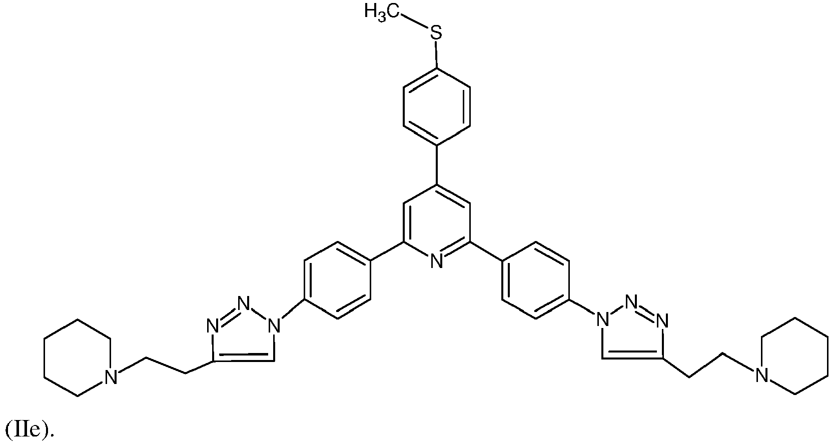

9. A combination comprising (i) at least one compound according to claims 1 to 8, and (ii) at least one lysosomotropic agent, in particular selected in the group comprising: chloroquine and derivatives thereof; Lys05; siramesine; GNS561; nanaomycin; siomycin A; helenalin; or a pharmaceutically acceptable salt thereof, and in particular is chloroquine; Lys05; or a pharmaceutically acceptable thereof, and in particular is chloroquine diphosphate or Lys05.

10. A pharmaceutical composition comprising (i) at least one compound according to claims 1 to 8, or at least one combination according to claim 9, and (ii) a pharmaceutically acceptable excipient or carrier.

11. Kit-of-parts comprising at least a first and a second containers, (i) the first container containing at least a first composition comprising at least one compound according to anyone of claims 1 to 8, and (ii) the second container containing at least a second composition comprising at least one lysosomotropic agent, in particular according to claim 9.

12. The compound according to claims 1 to 8, for use as a medicament.

13. The compound according to claims 1 to 8, the combination according to claim 9, the pharmaceutical composition according to claim 10, or the kit-of-parts according to claim 11, for use in a therapeutic method for preventing and/or treating a cancer disease, in particular a chemoresistant cancer disease.

14. The compound or combination or pharmaceutical composition or kit-of-parts for use according to claim 13, wherein said cancer disease is selected from the group consisting of: breast cancer; colon cancer; rectal cancer; endometrial cancer; gastric carcinoma; glioblastoma; hepatocellular carcinoma; cervical carcinoma; lung adeno-carcinoma; melanoma; medulloblastoma; ovarian carcinoma; osteosarcoma; pancreatic cancer; prostate cancer; acute myelogenous leukemia; chronic myelogenous leukemia; non-Hodgkin's lymphoma; thyroid carcinoma; and pediatric tumors.

15. The combination according to claim 9, the pharmaceutical composition according to claim 10, or the kit-of-parts according to claim 11, wherein said compound and said lysosomotropic agent are for simultaneous, separate or sequential use.

## Patentansprüche

1. Verbindung der allgemeinen Formel (I): 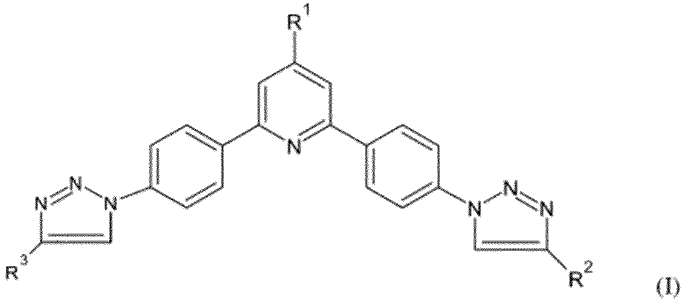 wobei
- R¹ ein 5- bis 6-gliedriger ungesättigter Ring ist, gegebenenfalls umfassend mindestens ein Heteroatom, und gegebenenfalls mit entweder mindestens einem der Folgenden substituiert ist:
- C₁-C₄ gesättigte oder ungesättigte, lineare oder verzweigte, Alkylgruppe;
- Y-R⁴-Gruppe, wobei Y N, O oder S ist und R⁴ entweder eine C₁-C₄, gesättigte oder ungesättigte, lineare oder verzweigte, Alkylgruppe, oder eine Benzylgruppe ist; oder
- einem Halogenatom, ausgewählt aus F, CI oder Br;
- R² und R³, verschieden oder identisch, insbesondere identisch, jeweils -(CH₂)ₙ-Het sind,
mit
- n in einem Bereich von 1 bis 4, insbesondere von 2 bis 3, liegt, und
- Het eine gesättigte oder ungesättigte 5- bis 6-gliedrige Heterozyklusgruppe ist, gegebenenfalls substituiert mit mindestens einer C₁-C₄ gesättigten oder ungesättigten, linearen oder verzweigten, Alkylgruppe;
oder ein Racemat, Enantiomer, Diastereomer einer solchen Verbindung oder einer Mischung davon,
oder eines Additionssalzes einer solchen Verbindung, Racemats, Enantiomers, Diastereomers oder einer Mischung mit einer Mineralsäure oder organischen Säure.

2. Verbindung nach Anspruch 1, wobei:
- R¹ ein 5- bis 6-gliedriger ungesättigter Ring ist, gegebenenfalls umfassend mindestens ein O oder S, und gegebenenfalls mit mindestens einer Gruppe der Folgenden substituiert ist:
- Y-R⁴, wobei Y O oder S ist und R⁴ entweder eine C₁-C₃, gesättigte oder ungesättigte, lineare oder verzweigte, Alkylgruppe oder eine Benzylgruppe ist; oder
- CI oder Br;
- R² und R³, verschieden oder identisch, insbesondere identisch, jeweils -(CH₂)ₙ-Het sind,
mit
- n 1, 2 oder 3, insbesondere 2 oder 3, ist, und
- Het eine gesättigte oder ungesättigte 5- bis 6-gliedrige Stickstoffheterozyklusgruppe ist, gegebenenfalls substituiert mit mindestens einer C₁-C₃ gesättigten oder ungesättigten, linearen oder verzweigten, Alkylgruppe.

3. Verbindung nach einem der Ansprüche 1 oder 2, wobei R¹ entweder ein unsubstituierter, ungesättigter 5- bis 6-gliedriger Ring ist, umfassend ein O oder S, oder eine Phenylgruppe ist, die mit mindestens einer Gruppe ausgewählt aus Y-R⁴, CI oder Br, substituiert ist, wobei Y O oder S ist und R⁴ entweder eine C₁-C₃, gesättigte oder ungesättigte, lineare oder verzweigte Alkylgruppe oder eine Benzylgruppe ist, und insbesondere R¹ ausgewählt ist aus einem Furyl; einem Thienyl; oder einem Phenyl, das mit mindestens einer Gruppe ausgewählt aus Y-R⁴, CI oder Br substituiert ist, wobei Y O oder S ist und R⁴ entweder eine C₁-C₂, gesättigte oder ungesättigte, Alkylgruppe oder eine Benzylgruppe ist.

4. Verbindung nach einem der vorhergehenden Ansprüche, wobei R¹ ein Phenyl ist, das mit mindestens einer Gruppe substituiert ist, insbesondere mit einer einzigen substituierenden Gruppe substituiert ist, insbesondere an Position 4 des Phenyls, wobei die Gruppe Y-R⁴ oder Br ist, wobei Y O oder S ist und R⁴ entweder eine Methyl- oder eine Benzylgruppe ist.

5. Verbindung nach einem der vorhergehenden Ansprüche, wobei R¹ ein Phenyl ist, das mit mindestens einer Gruppe substituiert ist, insbesondere mit einer einzelnen substituierenden Gruppe substituiert ist, insbesondere an Position 4 des Phenyls, wobei die Gruppe S-CH₃, O-Benzyl, oder Br, ist, und insbesondere S-CH₃ ist.

6. Verbindung nach einem der vorhergehenden Ansprüche, wobei Het eine gesättigte 5- bis 6-gliedrige Stickstoffheterozyklusgruppe ist, umfassend ein oder zwei Stickstoffatome, insbesondere Het ausgewählt ist aus einer Pyrrolidinyl-, einer Piperidinyl- oder einer Piperazinylgruppe, insbesondere Het nicht substituiert ist oder mit mindestens einer C₁-C₂, gesättigten oder ungesättigten, Alkylgruppe substituiert ist, und insbesondere Het mit mindestens einer Methylgruppe substituiert ist.

7. Verbindung nach einem der vorhergehenden Ansprüche, wobei Het entweder ein unsubstituiertes Pyrrolidin oder Piperidin ist oder ein Piperazin, das mit einer Methylgruppe an Position 4 substituiert ist.

8. Verbindung nach einem der vorhergehenden Ansprüche, die ausgewählt ist aus der Gruppe bestehend aus Formeln (IIa), (IIb), (IIc), (IId) oder (IIe):

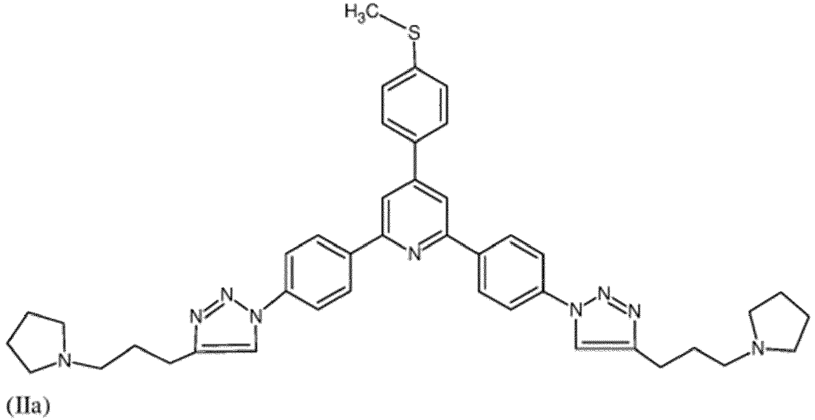

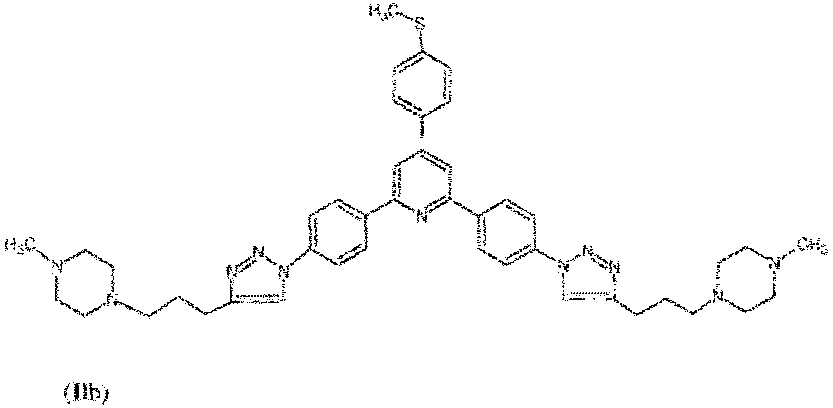

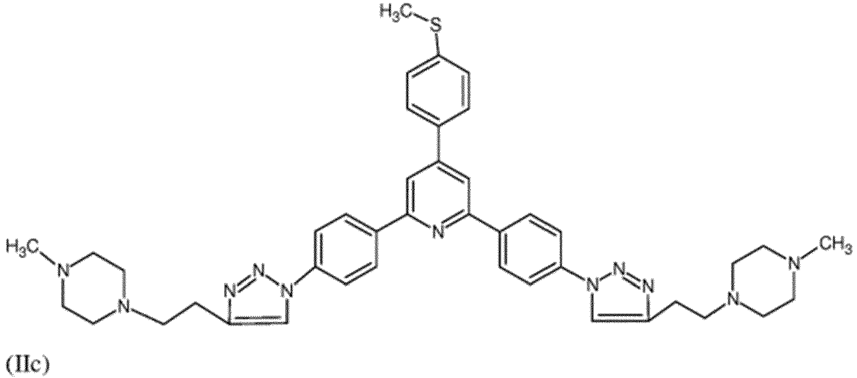

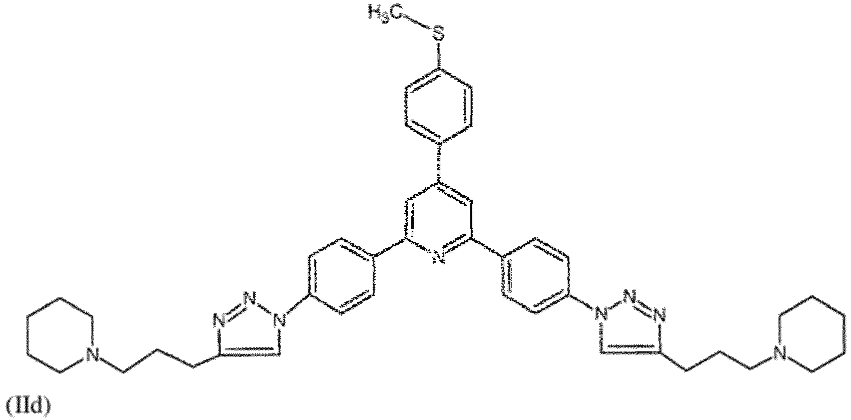

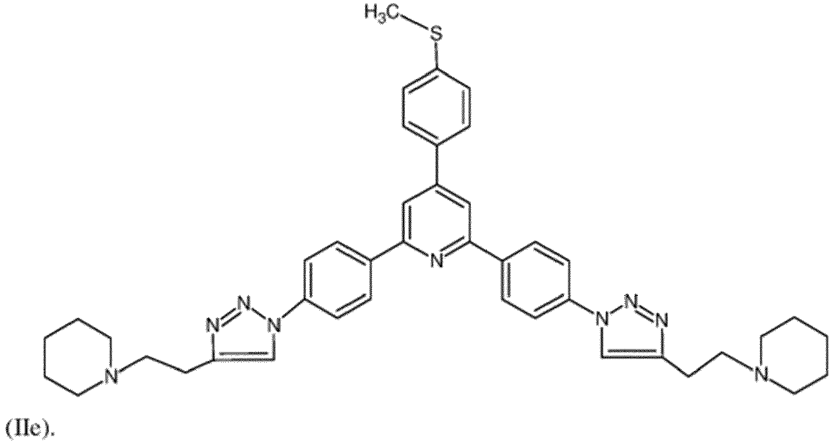

9. Kombination, umfassend (i) mindestens eine Verbindung nach Ansprüche 1 bis 8, und (ii) mindestens ein lysosomotropes Mittel, insbesondere ausgewählt aus der Gruppe, umfassend: Chloroquin und Derivate davon; Lys05; Siramesin; GNS561; Nanaomycin; Siomycin A; Helenalin; oder ein pharmazeutisch akzeptables Salz davon, und insbesondere ist es Chloroquin; Lys05; oder ein pharmazeutisch akzeptables davon, und insbesondere ist es Chloroquin-Diphosphat oder Lys05.

10. Pharmazeutische Zusammensetzung, umfassend (i) mindestens eine Verbindung nach den Ansprüchen 1 bis 8 oder mindestens eine Kombination nach Anspruch 9, und (ii) einen pharmazeutisch akzeptablen Hilfsstoff oder Träger.

11. Baukastensystem, umfassend mindestens einen ersten und einen zweiten Behälter, wobei (i) der erste Behälter mindestens eine erste Zusammensetzung enthält, umfassend mindestens eine Verbindung nach einem der Ansprüche 1 bis 8, und (ii) der zweite Behälter mindestens eine zweite Zusammensetzung enthält, umfassend mindestens ein lysosomotropes Mittel, insbesondere nach Anspruch 9.

12. Verbindung nach Ansprüche 1 bis 8, zur Verwendung als ein Medikament.

13. Verbindung nach einem der Ansprüche 1 bis 8, Kombination nach Anspruch 9, pharmazeutische Zusammensetzung nach Anspruch 10, oder Baukastensystem nach Anspruch 11, zur Verwendung in einem therapeutischen Verfahren zur Vorbeugung und/oder Behandlung einer Krebserkrankung, insbesondere einer chemoresistenten Krebserkrankung.

14. Verbindung oder Kombination oder pharmazeutische Zusammensetzung oder Baukastensystem zur Verwendung nach Anspruch 13, wobei die Krebserkrankung ausgewählt ist aus der Gruppe bestehend aus: Brustkrebs; Dickdarmkrebs; Mastdarmkrebs; Endometriumkrebs; Magenkarzinom; Glioblastom; hepatozellulärem Karzinom; Zervixkarzinom; Lungen-Adenokarzinom; Melanom; Medulloblastom; Ovarialkarzinom; Osteosarkom; Bauchspeicheldrüsenkrebs; Prostatakrebs; akuter myeloischer Leukämie; chronischer myeloischer Leukämie; Non-Hodgkin-Lymphom; Schilddrüsenkarzinom; und pädiatrischen Tumoren.

15. Kombination nach Anspruch 9, pharmazeutische Zusammensetzung nach Anspruch 10, oder Baukastensystem nach Anspruch 11, wobei die Verbindung und das lysosomotrope Mittel zur gleichzeitigen, getrennten oder sequenziellen Verwendung dienen.

## Revendications

1. Composé de formule générale (I) : 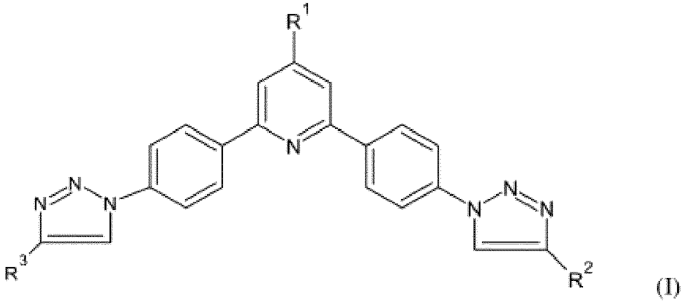 dans lequel
- R¹ est un cycle insaturé de 5 à 6 chaînons, comprenant optionnellement au moins un hétéroatome, et optionnellement substitué avec au moins l'un quelconque de :
- un groupe alkyle, linéaire ou ramifié, saturé ou insaturé, en C₁-C₄ ;
- un groupe en Y-R⁴, Y étant N, O ou S et R⁴ étant soit un groupe alkyle, linéaire ou ramifié, saturé ou insaturé, en C₁-C₄, soit un groupe benzyle ; ou
- un atome d'halogène sélectionné parmi F, Cl ou Br ;
- R² et R³, différents ou identiques, en particulier identiques, sont chacun -(CH₂)ₙ-Het, avec
- n allant de 1 à 4, en particulier de 2 à 3, et
- Het étant un groupe hétérocyclique de 5 à 6 chaînons saturé ou insaturé, optionnellement substitué avec au moins un groupe alkyle, linéaire ou ramifié, saturé ou insaturé en C₁-C₄ ;
ou un racémate, un énantiomère, un diastéréo-isomère d'un tel composé ou mélange de ceux-ci, ou un sel d'ajout d'un tel composé, racémate, énantiomère, diastéréo-isomère ou un mélange avec un acide minéral ou acide organique.

2. Composé selon la revendication 1, dans lequel :
- R¹ est un cycle insaturé de 5 à 6 chaînons, comprenant optionnellement au moins un O ou S, et optionnellement substitué avec au moins un groupe, qui est :
- Y-R⁴, Y étant N, O ou S et R⁴ étant soit un groupe alkyle, linéaire ou ramifié, saturé ou insaturé, en C₁-C₃, soit un groupe benzyle ; ou
- Cl ou Br ;
- R² et R³, différents ou identiques, en particulier identiques, sont chacun -(CH₂)ₙ-Het, avec
- n étant 1, 2 ou 3, en particulier 2 ou 3, et
- Het étant un groupe hétérocyclique d'azote de 5 à 6 chaînons saturé ou insaturé, optionnellement substitué avec au moins un groupe alkyle, linéaire ou ramifié, saturé ou insaturé en C₁-C₃.

3. Composé selon l'une quelconque des revendications 1 ou 2, dans lequel R¹ est soit un cycle de 5 à 6 chaînons insaturé non substitué, comprenant un O ou S, ou est un groupe phényle substitué avec au moins un groupe sélectionné parmi Y-R⁴, Cl ou Br, Y étant O ou S et R⁴ étant soit un groupe alkyle, linéaire ou ramifié, saturé ou insaturé, en C₁-C₃, soit un groupe benzyle, et en particulier R¹ est sélectionné parmi un furyle ; un thiényle ; ou un phényle substitué avec au moins un groupe sélectionné parmi Y-R⁴, Cl ou Br, dans lequel Y est O ou S et R⁴ est soit un groupe alkyle, saturé ou insaturé, en C₁-C₂, soit un groupe benzyle.

4. Composé selon l'une quelconque des revendications précédentes, dans lequel R¹ est un phényle substitué avec au moins un groupe, en particulier est substitué avec un unique groupe de substitution, en particulier en position 4 du phényle, ledit groupe étant Y-R⁴ ou Br, Y étant O ou S et R⁴ étant soit un méthyle, soit un groupe benzyle.

5. Composé selon l'une quelconque des revendications précédentes, dans lequel R¹ est un phényle substitué avec au moins un groupe, est en particulier substitué avec un unique groupe de substitution, en particulier en position 4 du phényle, ledit groupe étant S-CH₃, O-benzyle, ou Br, et en particulier étant S-CH₃.

6. Composé selon l'une quelconque des revendications précédentes, dans lequel Het est un groupe hétérocyclique d'azote de 5 à 6 chaînons comprenant un ou deux atomes d'azote, en particulier Het est sélectionné parmi un groupe pyrrolidinyle, pipéridinyle, ou pipérazinyle, en particulier Het est non substitué ou est substitué avec au moins un groupe alkyle, saturé ou insaturé, en C₁-C₂, et Het est en particulier substitué avec au moins un groupe méthyle.

7. Composé selon l'une quelconque des revendications précédentes, dans lequel Het est soit une pyrrolidine soit une pipéridine non substituée, ou est une pipérazine substituée avec un groupe méthyle en position 4.

8. Composé selon l'une quelconque des revendications précédentes, qui est sélectionné parmi le groupe constitué de formules (IIa), (IIb), (IIc), (IId) ou (IIe) :

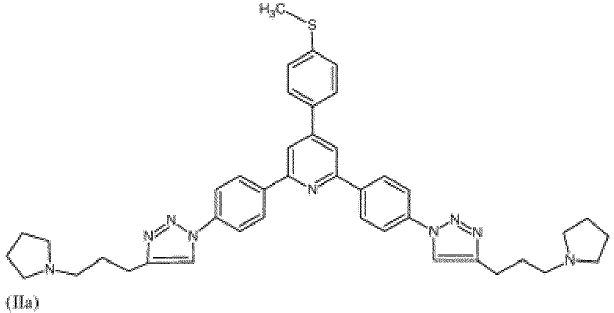

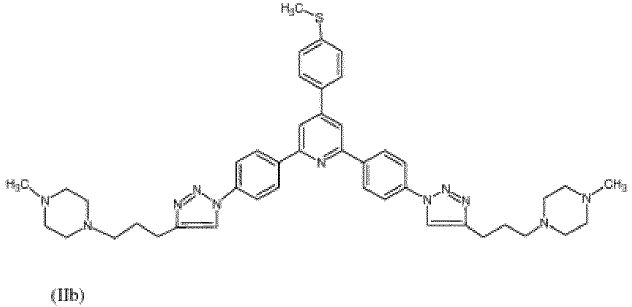

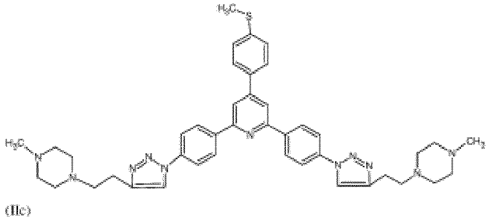

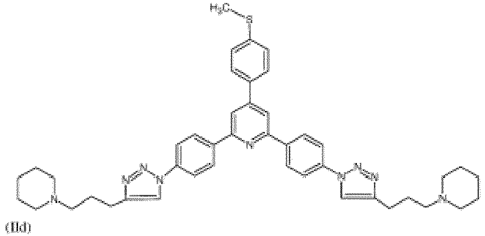

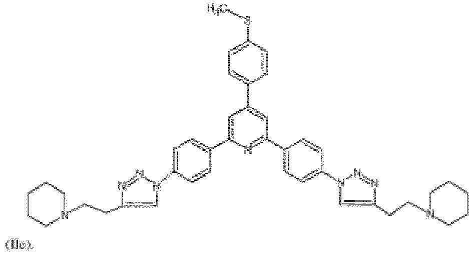

9. Combinaison comprenant (i) au moins un composé selon les revendications 1 à 8, et (ii) au moins un agent lysosomotrope, en particulier sélectionné dans le groupe comprenant : de la chloroquine et ses dérivés ; Lys05 ; de la siramésine ; GNS561 ; de la nanaomycine ; de la siomycine A ; de l'hélénaline ; ou un sel pharmaceutiquement acceptable de celles-ci, et en particulier de la chloroquine ; Lys05 ; ou un sel pharmaceutiquement acceptable de celles-ci, et en particulier du diphosphate de chloroquine ou Lys05.

10. Composition pharmaceutique comprenant (i) au moins un composé selon les revendications 1 à 8, ou au moins une combinaison selon la revendication 9, et (ii) un excipient ou vecteur pharmaceutiquement acceptable.

11. Kit de parties comprenant au moins des premier et second récipients, (i) le premier récipient contenant au moins une première composition comprenant au moins un composé selon l'une quelconque des revendications 1 à 8, et (ii) le second récipient contenant au moins une seconde composition comprenant au moins un agent lysosomotrope, en particulier selon la revendication 9.

12. Composé selon les revendications 1 à 8, pour son utilisation comme un médicament.

13. Composé selon les revendications 1 à 8, combinaison selon la revendication 9, composition pharmaceutique selon la revendication 10, ou kit de parties selon la revendication 11, pour son utilisation dans une méthode thérapeutique pour prévenir et/ou traiter une maladie cancéreuse, en particulier une maladie cancéreuse chimiorésistante.

14. Composé ou combinaison ou composition pharmaceutique ou kit de parties pour son utilisation selon la revendication 13, dans lequel/laquelle ladite maladie cancéreuse est sélectionnée parmi le groupe composé : du cancer du sein ; du cancer du côlon ; du cancer du rectum ; du cancer du col de l'utérus ; du carcinome gastrique ; du glioblastome ; du carcinome hépatocellulaire ; du carcinome cervical ; de l'adénocarcinome du poumon ; du mélanome ; du médulloblastome ; du carcinome ovarien ; de l'ostéosarcome ; du cancer du pancréas ; du cancer de la prostate ; de la leucémie myéloblastique aiguë ; de la leucémie myéloblastique chronique ; du lymphome non hodgkinien ; du carcinome de la thyroïde ; et de tumeurs pédiatriques.

15. Combinaison selon la revendication 9, composition pharmaceutique selon la revendication 10, ou kit de parties selon la revendication 11, dans lequel/laquelle ledit composé et ledit agent lysosomotrope sont destinés à une utilisation simultanée, séparée ou séquentielle.
